# EUROPÄISCHE PATENTSCHRIFT

(11) **EP 0 761 772 B1**
(45) Veröffentlichungstag und Bekanntmachung des Hinweises auf die Patenterteilung: **15.03.2000**
(21) Anmeldenummer: 96810476.0
(22) Anmeldetag: 19.07.1996
(51) Int. Cl.: C09B 69/08, C09B 1/00, C09B 7/02, C09B 29/036, C09B 5/62, C09B 19/02, C09B 47/26, C09B 57/04, C09B 7/00, C09B 48/00

(54) **Lösliche Chromophore mit leicht abspaltbaren löslichmachenden Gruppen**
Soluble chromophors containing solubility-enhancing groups which separate easily therefrom
Chromophores solubles portant des groupes solubilisants facilement séparables

(30) Priorität: 28.07.1995 CH 222295; 19.10.1995 CH 296895
(43) Veröffentlichungstag der Anmeldung: 12.03.1997
(73) Patentinhaber: Ciba Specialty Chemicals Holding Inc., 4057 Basel (CH)
(72) Erfinder: Hall-Goulle, Véronique, 3012 Bern (CH)

(56) Entgegenhaltungen:
- EP-A- 0 648 770
- EP-A- 0 648 817
- EP-A- 0 654 506

## Beschreibung

Die vorliegende Anmeldung betrifft neue lösliche Chromophore mit leicht abspaltbaren löslichmachenden ungesättigten Hydrocarbyloxycarbonylgruppen. Dank dieser Eigenschaft können diese Chromophore sehr leicht selbst im Substrat, in welchem sie problemlos in gelöster Form eingearbeitet werden können, zu den entsprechenden Pigmenten umgewandelt werden.

Aus EP-A 648 770 und 648 817 sind Carbamatgruppen enthaltende lösliche Chromophore bekannt, die durch Erhitzen auf höhere Temperaturen und daraus folgender Abspaltung der die Carbamatreste bildenden Hydrocarbyloxycarbonylgruppen in die entsprechenden Pigmente überführt werden können. Unter zahlreichen anderen werden generisch auch Alkenylcarbamatreste erwähnt.

Es wurde nun gefunden, dass lösliche Chromophore mit in β-Stellung zum Sauerstoff der Carbonyloxygruppe ungesättigten Carbamatresten gegenüber den nächstvergleichbaren bekannten Carbamatgruppen enthaltenden löslichen Chromophoren ohne diese Eigentümlichkeit ganz überraschend bei deutlich tieferen Temperaturen, d.h. mit einem beachtlich geringeren Energieaufwand, in die entsprechenden Pigmente überführt werden können.

Die vorliegende Erfindung betrifft demnach Verbindungen der Formel

A(B)ₓ (I),

worin
x eine ganze Zahl zwischen 1 und 4 bedeutet,
A für den Rest eines Chromophors der Chinacridon-, Anthrachinon-, Perylen-, Indigo-, Chinophthalon-, Isoindolinon-, Isoindolin-, Dioxazin, Phthalocyanin, Diketopyrrolopyrrol- oder Azoreihe steht, der x mit B verbundene N-Atome, vorzugsweise mit mindestens einer unmittelbar benachbarten oder konjugierten Carbonylgruppe, enthält,
B für eine Gruppe der Formel 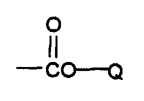 steht, und, wenn x 2, 3 oder 4 bedeutet, auch ein-, zwei- oder dreimal Wasserstoff sein kann, wobei Q eine Gruppe der Formel 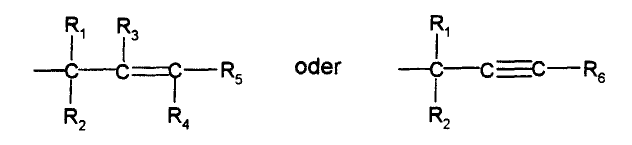 ist, worin
R₁ und R₂ unabhängig voneinander Wasserstoff, C₁-C₂₄- Alkyl, durch O, S oder N(R₉)₂ unterbrochenes C₁-C₂₄-Alkyl, C₃-C₂₄-Alkenyl, C₃-C₂₄-Alkinil, C₄-C₁₂-Cycloalkyl, C₄-C₁₂-Cycloalkenyl, unsubstituiertes oder durch C₁-C₆-Alkyl, C₁-C₆-Alkoxy, Halogen, Cyano oder Nitro substituiertes Phenyl oder Biphenyl bedeuten,
R₃, R₄ und R₅ unabhängig voneinander Wasserstoff, C₁-C₂₄-Alkyl oder C₃-C₂₄-Alkenyl sind,
R₆ Wasserstoff, C₁-C₂₄-Alkyl, C₃-C₂₄-Alkenyl oder eine Gruppe der Formel 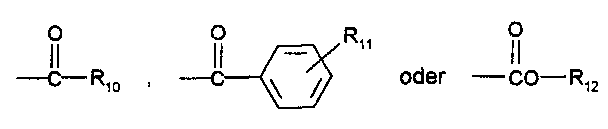 ist,
R₉ und R₁₀ C₁-C₆-Alkyl, R₁₁ Wasserstoff oder C₁-C₆-Alkyl und R₁₂ Wasserstoff, C₁-C₆-Alkyl, unsubstituiertes oder durch C₁-C₆-Alkyl substituiertes Phenyl sind,
mit der Massgabe, dass wenn A für den Rest eines Chromophors der Diketopyrrolopyrrolreihe steht, Q keine Gruppe der Formel 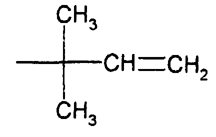 ist.

A bedeutet den Rest bekannter Chromophore mit der Grundstruktur

A(H)ₓ,

wie beispielsweise 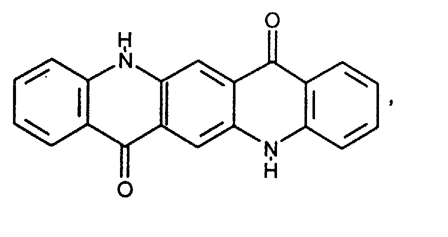 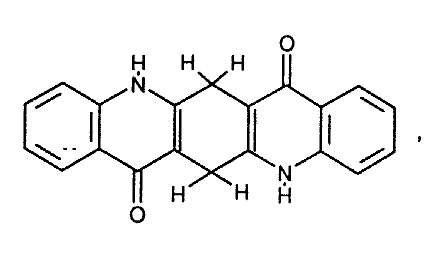 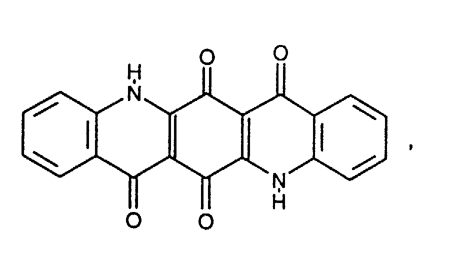 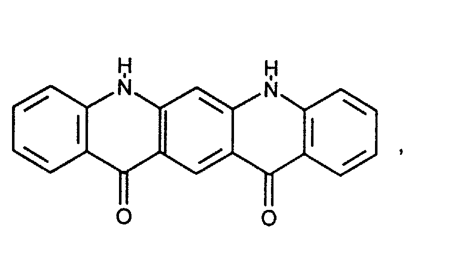 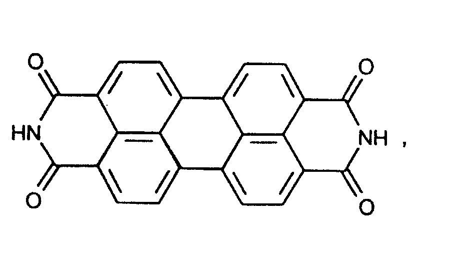 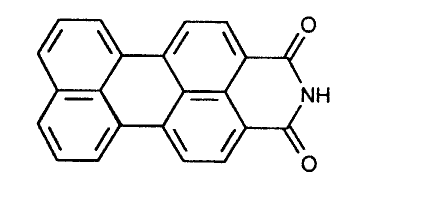 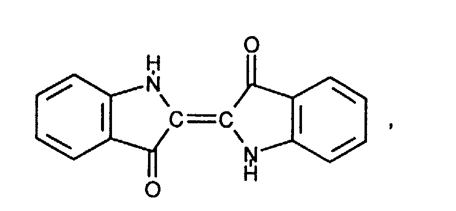 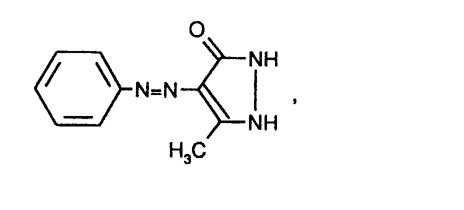 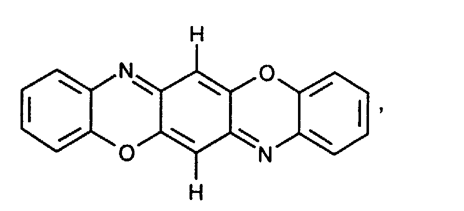 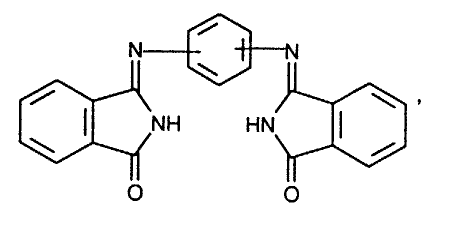 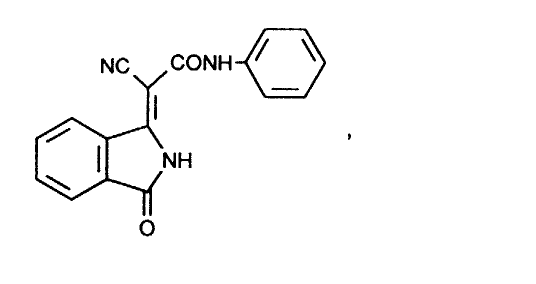 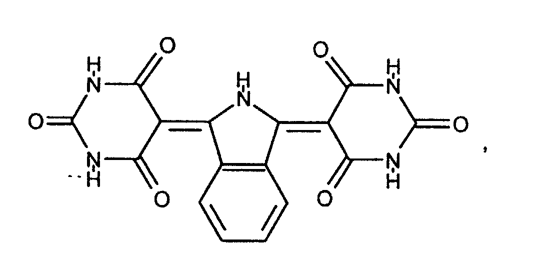 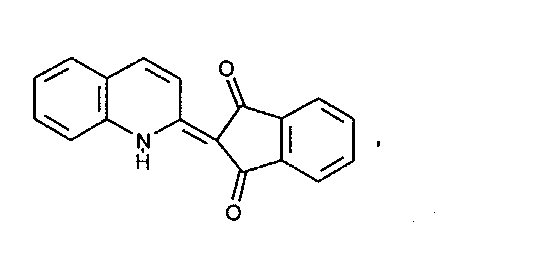 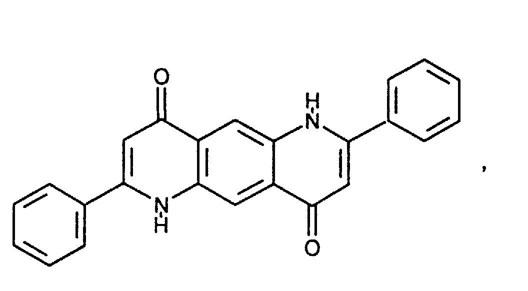 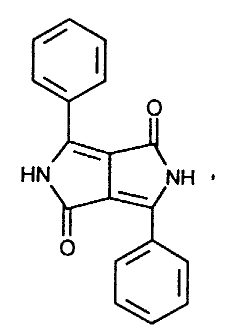 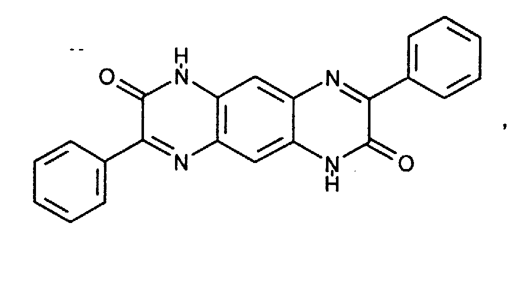 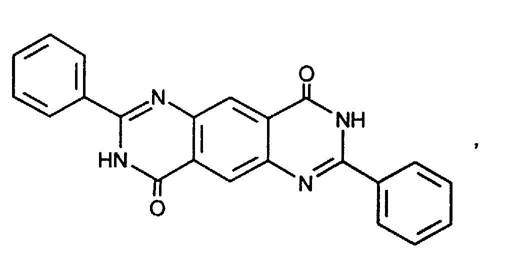 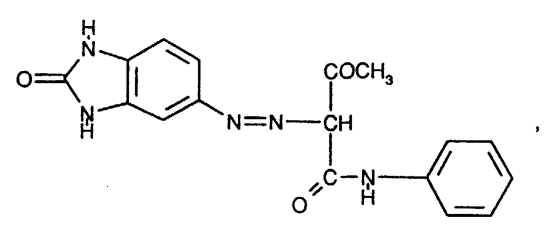 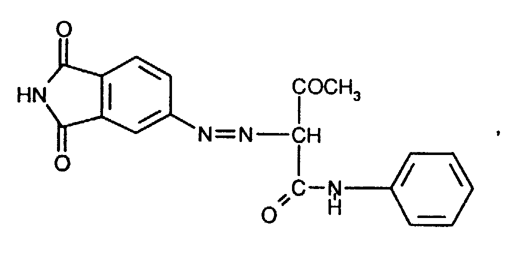 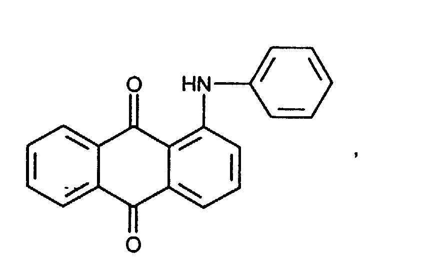 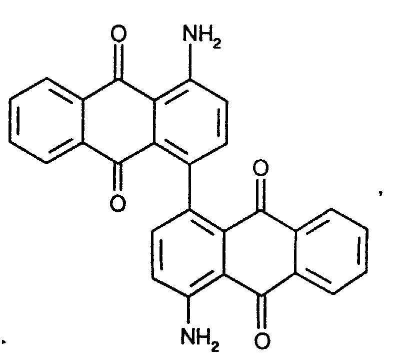 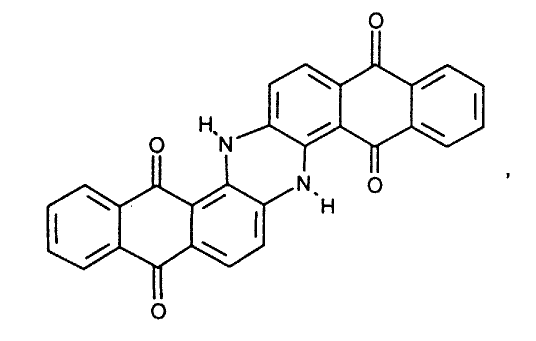 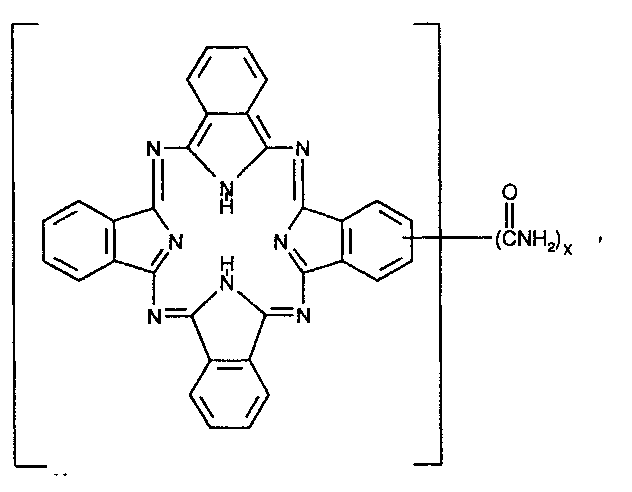 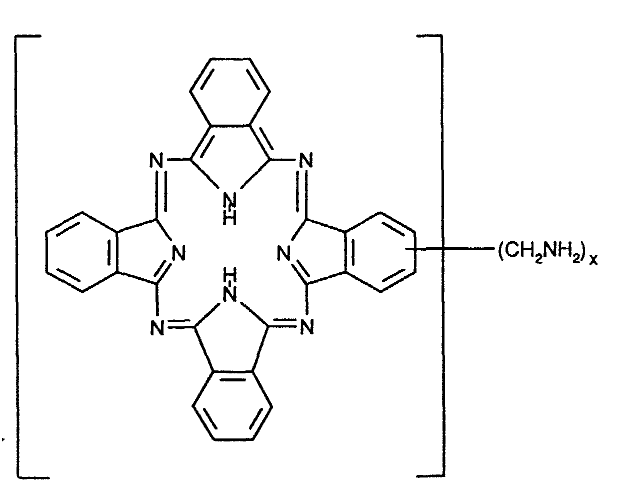 und jeweils alle bekannten Derivate davon.

Bedeuten etwaige Substituenten C₁-C₆-Alkyl, so handelt es sich z.B. um Methyl, Ethyl, n-Propyl, Isopropyl, n-Butyl, sec.-Butyl, tert.-Butyl, n-Pentyl, tert.-Amyl, n-Hexyl, oder 2,2-Dimetylbutyl. C₁-C₂₄-Alkyl kann darüberhinaus beispielsweise n-Octyl, 1,1,3,3-Tetramethylbutyl, 2-Ethylhexyl, Nonyl, Decyl, Dodecyl, Tetradecyl, Hexadecyl, Octadecyl, Eicosyl, Heneicosyl, Docosyl oder Tetracoxyl bedeuten.

C₃-C₂₄-Alkenyl ist C₃-C₂₄-Alkyl, welches ein- oder mehrfach ungesättigt ist, wobei zwei oder mehr Doppelbindungen gegebenenfalls isoliert oder konjugiert sein können, zum Beispiel Allyl, 2-Propen-2-yl, 2-Buten-1-yl, 3-Buten-1-yl, 1,3-Butadien-2-yl, 2-Penten-1-yl, 3-Penten-2-yl, 2-Methyl-1-buten-3-yl, 2-Methyl-3-buten-2-yl, 3-Methyl-2-buten-1-yl, 1,4-Pentadien-3-yl, oder die verschiedenen Isomeren von Hexenyl, Octenyl, Nonenyl, Decenyl, Dodecenyl, Tetradecenyl, Hexadecenyl, Octadecenyl, Eicosenyl, Heneicosenyl, Docosenyl, Tetracosenyl, Hexadienyl, Octadienyl, Nonadienyl, Decadienyl, Dodecadienyl, Tetradecadienyl, Hexadecadienyl, Octadecadienyl, Eicosadienyl, Heneicosadienyl, Docosadienyl oder Tetracosadienyl.

C₄-C₁₂-Cycloalkyl ist zum Beispiel ein monozyklisches Cycloalkyl, wie beispielsweise Cyclobutyl, Cyclopentyl, Cyclohexyl, Trimethylcyclohexyl oder Menthyl, oder ein polyzyklisches Cycloalkyl, wie beispielsweise Thujyl, Bornyl, 1-Adamantyl oder 2-Adamantyl.

C₄-C₁₂-Cycloalkenyl ist C₄-C₁₂-Cycloalkyl, welches ein- oder mehrfach ungesättigt ist, wobei zwei oder mehr Doppelbindungen gegebenenfalls isoliert oder konjugiert sein können, zum Beispiel 2-Cyclobuten-1-yl, 2-Cyclopenten-1-yl, 2-Cyclohexen-1-yl, 3-Cyclohexen-1-yl, 2,4-Cyclohexadien-1-yl, 1-*p*-Menthen-8-yl, 4(10)-Thujen-10-yl, 2-Norbornen-1-yl, 2,5-Norbomadien-1-yl oder 7,7-Dimethyl-2,4-norcaradien-3-yl.

C₃-C₂₄-Alkinyl ist C₃-C₂₄-Alkyl oder C₃-C₂₄-Alkenyl, welches ein- oder mehrfach doppelt ungesättigt ist, wobei die Dreifachbindungen gegebenenfalls isoliert oder unter sich oder mit Doppelbindungen konjugiert sein können, zum Beispiel 1-Propin-3-yl, 1-Butin-4-yl, 1-Pentin-5-yl, 2-Methyl-3-butin-2-yl, 1,4-Pentadiin-3-yl, 1,3-Pentadiin-5-yl, 1-Hexin-6-yl, cis-3-Methyl-2-penten-4-in-1-yl, trans-3-Methyl-2-penten-4-in-1-yl, 1,3-Hexadiin-5-yl, 1-Octin-8-yl, 1-Nonin-9-yl, 1-Decin-10-yl oder 1-Tetracosin-24-yl.

C₁-C₆-Alkoxy bedeutet z.B. Methoxy, Ethoxy, n-Propoxy, Isopropoxy, n-Butoxy, tert.-Butoxy, n-Amyloxy, tert.-Amyloxy oder n-Hexyloxy.

Halogen bedeutet z.B. Jod, Fluor, Brom oder insbesondere Chlor.

Von besonderem Interesse sind Verbindungen der Formel I, worin R₁ C₁-C₂₄-Alkyl, C₃-C₂₄-Alkenyl, C₃-C₂₄-Alkinyl, unsubstituiertes oder durch C₁-C₆-Alkyl, C₁-C₆-Alkoxy, Halogen, Cyano oder Nitro substituiertes Phenyl und R₆ C₁-C₂₄-Alkyl oder C₃-C₂₄-Alkenyl bedeuten, insbesondere jene, worin
R₁ und R₂ Methyl sind,
R₃, R₄ und R₅ unabhängig voneinander Wasserstoff oder C₁-C₁₂-Alkyl bedeuten,
R₆ C₁-C₆-Alkyl ist
und ganz besonders diejenigen, worin
B eine Gruppe der Formel 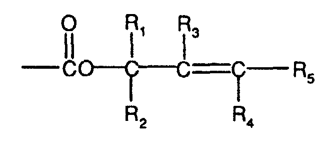 ist,
R₁ und R₂ Methyl und R₃, R₄ und R₅ unabhängig voneinander Wasserstoff oder C₁-C₁₂-Alkyl bedeuten.

Bevorzugte Verbindungen der Formel I sind:
a) Perylencarbonsäureimide der Formel 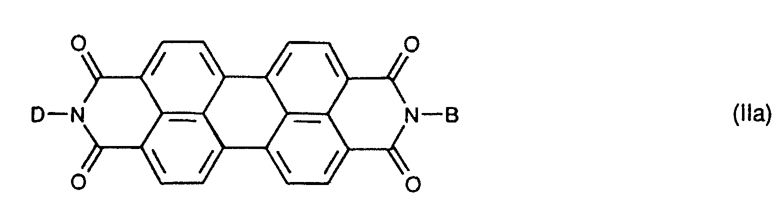 oder 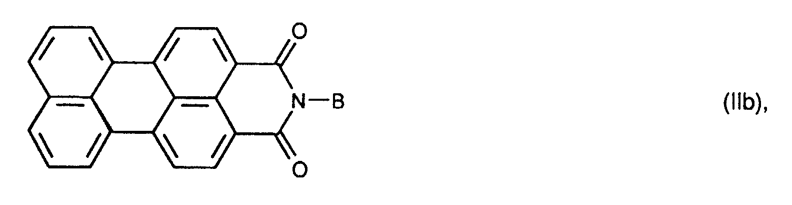 worin D Wasserstoff, C₁-C₆-Alkyl, unsubstituiertes oder durch Halogen oder C₁-C₆-Alkyl substituiertes Phenyl, Benzyl oder Phenethyl oder B bedeutet,
b) Chinacridone der Formel 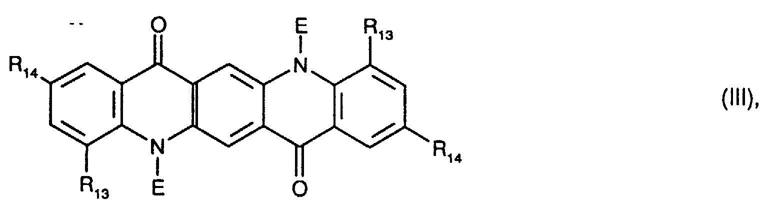 worin R₁₃ und R₁₄ unabhängig voneinander Wasserstoff, Halogen, C₁-C₂₄-Alkyl, C₁-C₆-Alkoxy oder Phenyl ist,
c) Dioxazine der Formel 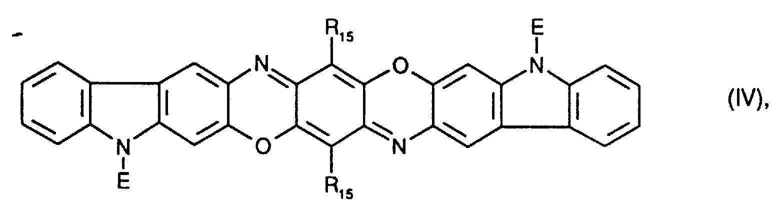 worin R₁₅ Wasserstoff, Halogen oder C₁-C₂₄-Alkyl ist,
   oder der Formel 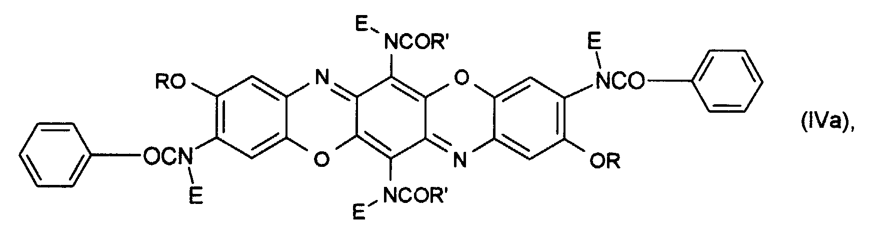 worin R und R' unabhängig voneinander C₁-C₄-Alkyl sind,
d) Isoindoline der Formeln 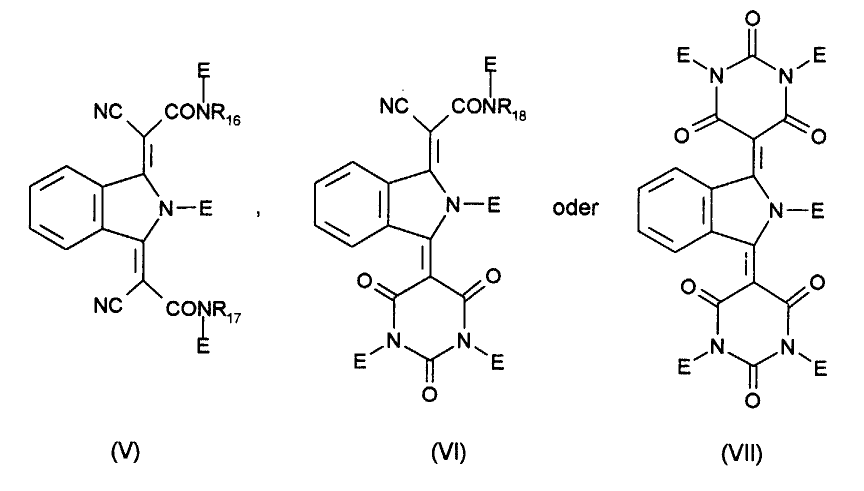 worin R₁₆ eine Gruppe 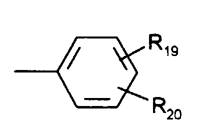 ist,
   R₁₇ Wasserstoff, C₁-C₂₄-Aklyl, Benzyl oder eine Gruppe 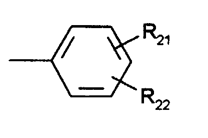 bedeutet
   R₁₈ die gleiche Bedeutung wie R₁₆ hat,
   R₁₉, R₂₀, R₂₁ und R₂₂ unabhängig voneinander Wasserstoff, C₁-C₂₄-Alkyl, C₁-C₆-Alkoxy, Halogen oder Trifluormethyl bedeuten,
e) Indigoderivate der Formel 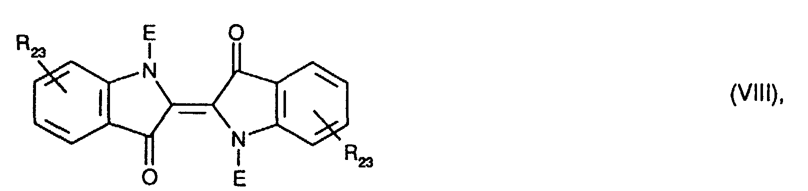 worin R₂₃ Wasserstoff, CN, C₁-C₆-Alkyl, C₁-C₆-Alkoxy oder Halogen bedeutet,
f) Benzimidazolon-Azoverbindungen der Formel 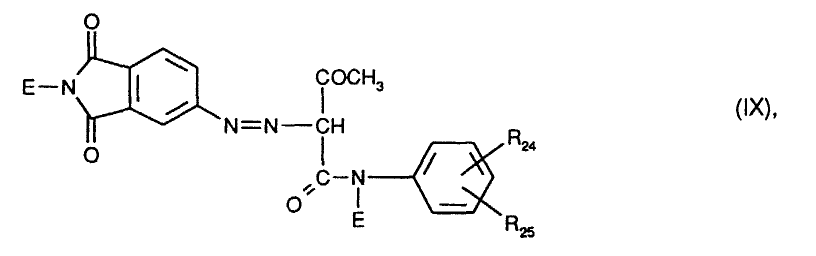 worin R₂₄ und R₂₅ unabhängig voneinander Wasserstoff, Halogen, C₁-C₆-Alkyl oder C₁-C₆-Alkoxy sind,
g) anthrachinoide Verbindungen der Formel 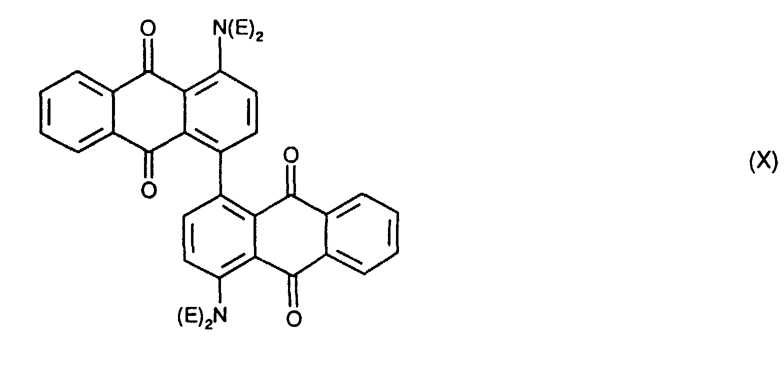 oder 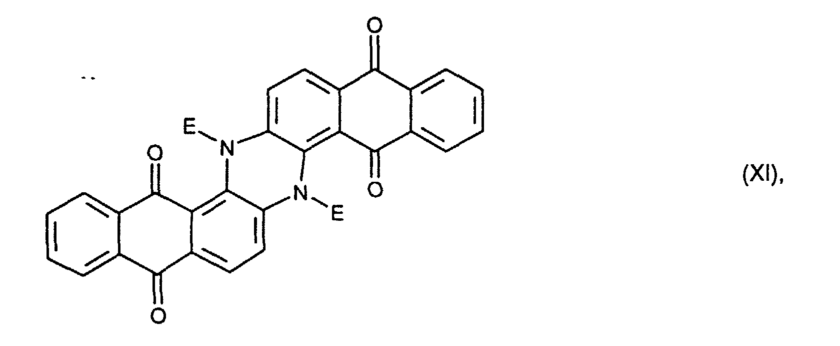
h) Phthalocyanine der Formel 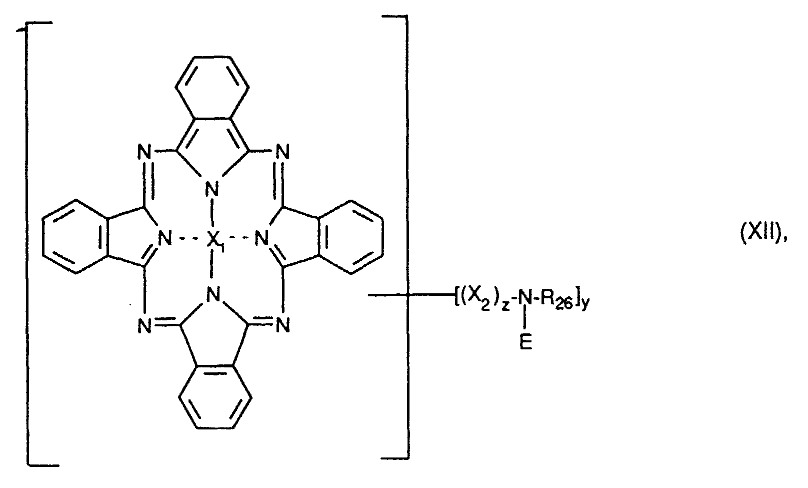 worin
   X₁ H₂, ein zweiwertiges Metall ausgewählt aus der Gruppe Cu(II), Zn(II), Fe(II), Ni(II), Ru(II), Rh(II), Pd(II), Pt(II), Mn(II), Mg(II), Be(II), Ca(II), Ba(II), Cd(II), Hg(II), Sn(II), Co(II) oder Pb(II) bevorzugt Cu(II), Zn(II), Fe(II) Ni(II) oder Pd(II) oder ein zweiwertiges Oxometall ausgewählt aus der Gruppe V(O), Mn(O) oder TiO,
   X₂-CH(R₂₄)-, -CO- oder -SO₂-
   R₂₆ Wasserstoff, C₁-C₆-Alkyl, -N(E)R₂₇, -NHCOR₂₈, -COR₂₈ oder 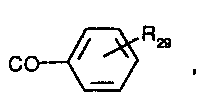
   R₂₇ Wasserstoff oder C₁-C₆-Alkyl, R₂₈ C₁-C₆-Alkyl und R₂₉ Wasserstoff, Halogen, C₁-C₆-Alkyl oder C₁-C₆-Alkoxy bedeuten,
   z für Null oder 1 und y für eine Zahl von 1 bis 4 stehen,
i) Isoindolinone der Formel 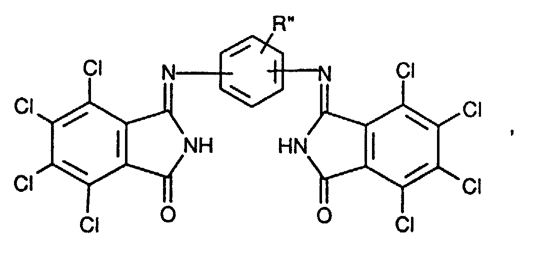 worin R" Wasserstoff oder C₁-C₄-Alkyl ist,
   und
j) Pyrrolo[3,4-c]pyrrole der Formel 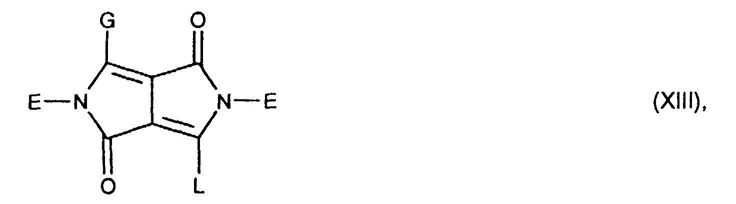
   worin G und L unabhängig voneinander für eine Gruppe der Formel 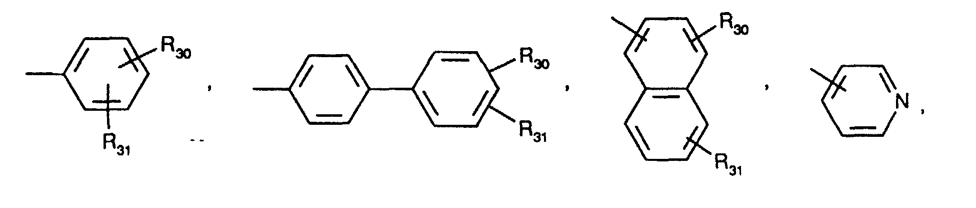 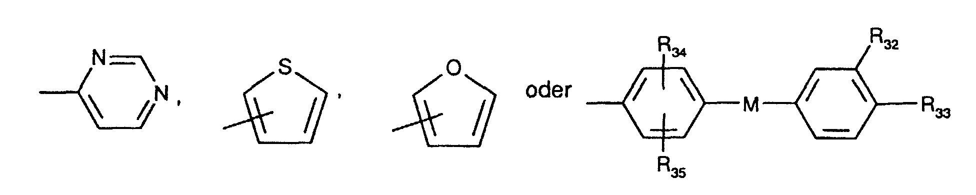 stehen, worin
   R₃₀ und R₃₁ unabhängig voneinander Wasserstoff, Halogen, C₁-C₂₄-Alkyl, C₁-C₆-Alkoxy, C₁-C₁₈-Alkylmercapto, C₁-C₁₈-Alkylamino, -CN, -NO₂, -Phenyl, Trifluormethyl, C₅-C₆-Cycloalkyl, -C=N-(C₁-C₂₄-Alkyl), 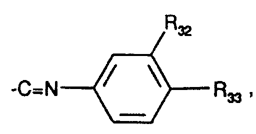 Imidazolyl, Pyrrazolyl, Triazolyl, Piperazinyl, Pyrrolyl, Oxazolyl, Benzoxazolyl, Benzthiazolyl, Benzimidazolyl, Morpholinyl, Piperidinyl oder Pyrrolidinyl bedeuten,
   M -CH₂-, -CH(CH₃)-, -C(CH₃)₂-, -CH=N-, -N=N-, -O-, -S-, -SO-, -SO₂- oder -NR₃₆- ist,
   R₃₂ und R₃₃ unabhängig voneinander Wasserstoff, Halogen, C₁-C₆-Alkyl, C₁-C₆-Alkoxy oder -CN sind, R₃₄ und R₃₅ unabhängig voneinander Wasserstoff, Halogen oder C₁-C₆-Alkyl und
   R₃₆ Wasserstoff oder C₁-C₆-Alkyl bedeuten,
   wobei in den obenerwähnten Formeln E jeweils Wasserstoff oder B bedeutet, mit der Massgabe, dass E in jeder Formel mindestens einmal B ist, und B die oben angegebene Bedeutung hat, für welche die obenerwähnten Bevorzugungen gelten.

Für R₃₀ und R₃₁ in Formel XIII stehen C₁-C₁₈-Alkylmercapto beispielsweise für Methylmercapto, Ethylmercapto, Propylmercapto, Butylmercapto, Octylmercapto, Decylmercapto, Hexydecylmercapto oder Octadecylmercapto und C₁-C₁₈-Alkylamino z.B. für Methylamino, Ethylamino, Propylamino, Hexylamino, Decylamino oder Octadecylamino.

Besonders bevorzugt sind Chinacridone der Formel III, worin R₁₃ und R₁₄ unabhängig voneinander Wasserstoff, Chlor oder Methyl bedeuten, oder Pyrrolo[3,4-c]pyrrole der Formel XIII, worin G und L gleich sind und eine Gruppe der Formel 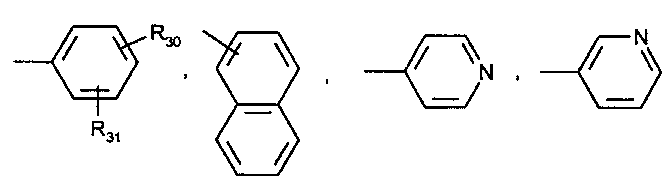 oder 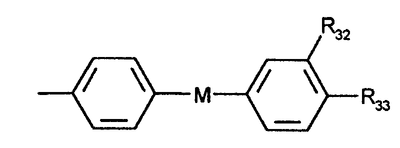 sind,
worin R₃₀ und R₃₁ unabhängig voneinander Wasserstoff, Chlor, Brom, C₁-C₄-Alkyl, C₁-C₆-Alkoxy, C₁-C₆-Alkylamino, CN oder Phenyl bedeuten,
M -O-, -NR₃₆-, -N=N- oder -SO₂- ist,
R₃₂ und R₃₃ Wasserstoff und R₃₆ Wasserstoff, Methyl oder Ethyl bedeuten, oder anthrachinoide Verbindungen der Formel Xl,
wobei E in den Formeln III, XIII und XI jeweils die oben angegebene Bedeutung hat.

Besonders bevorzugt sind Chinacridone der Formel 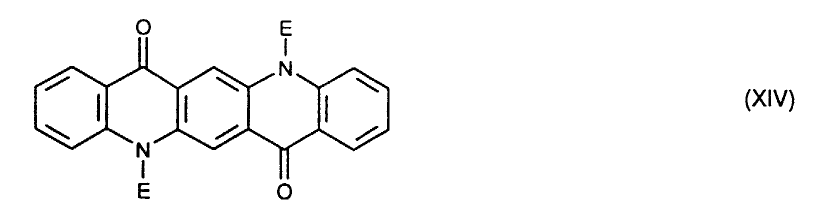 oder 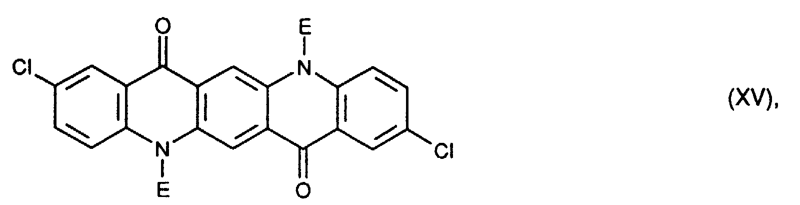 worin E Wasserstoff oder B bedeutet, mit der Massgabe, dass E in jeder Formel mindestens einmal B ist, und B die oben angegebene Bedeutung hat,
Pyrrolopyrrole der Formel 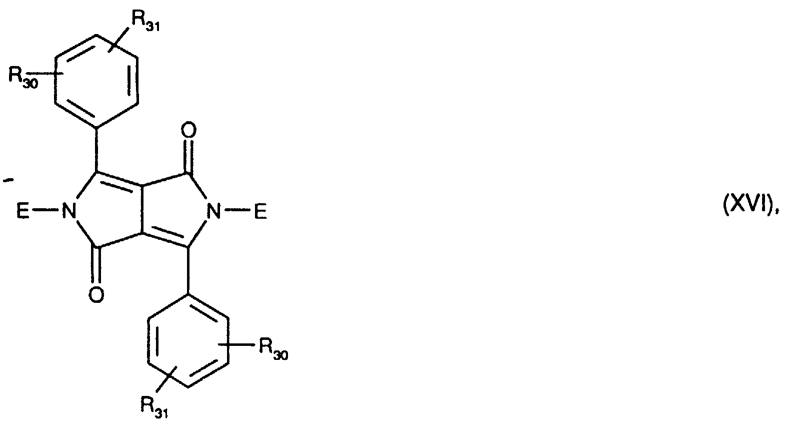 worin R₃₀ und R₃₁ unabhängig voneinander Wasserstoff, Methyl, tert.-Butyl, Chlor, Brom, CN oder Phenyl bedeuten, und E Wasserstoff oder B bedeutet, mit der Massgabe, dass E mindestens einmal B ist, und B die oben angegebene Bedeutung hat, und
anthrachinoide Verbindungen der Formel 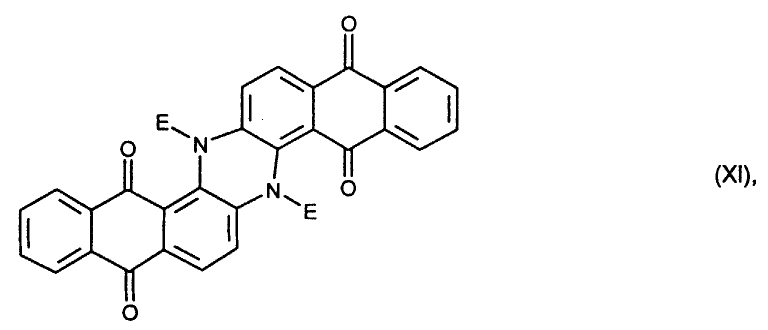 worin E Wasserstoff oder B bedeutet, mit der Massgabe, dass E mindestens einmal B ist, und B die oben angegebene Bedeutung hat.

Die Verbindungen der Formel I können in Analogie zu an sich bekannten Methoden, wie beispielsweise in EP-A 648 770 und EP-A 648 817 beschrieben, hergestellt werden, z.B. dadurch, dass eine Verbindung der Formel

A(H)ₓ, (XVII)

worin A und x die oben angegebene Bedeutung haben, im gewünschten Molverhältnis mit einem Dicarbonat der Formel

B - O - B (XVIII)

oder mit einem Trihaloessigsäureester der Formel

(R₃₇)₃C-B (XIX),

oder mit einem Azid der Formel

BN₃ (XX),

oder mit einem Carbonat der Formel

B-OR₃₈ (XXI),

oder mit einem Alkylideniminooxyameisensäureester der Formel 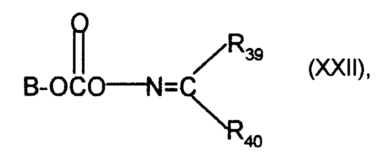 worin B jeweils die oben angegebene Bedeutung hat, R₃₇ Chlor, Fluor oder Brom, R₃₈ C₁-C₄-Alkyl oder unsubstituiertes oder durch Halogen, C₁-C₄-Alkyl, C₁-C₄-Alkoxy oder -CN substituiertes Phenyl, R₃₉ -CN oder -COOR₃₈ und R₄₀ unsubstituiertes oder durch Halogen, C₁-C₄-Alkyl, C₁-C₄-Alkoxy oder -CN substituiertes Phenyl bedeuten, in einem aprotischen organischen Lösungsmittel in Gegenwart einer Base als Katalysator, zweckmässig bei Temperaturen zwischen 0 und 120°C, bevorzugt zwischen 10 und 100°C, während 2 bis 80 Stunden, umgesetzt wird.

Bevorzugt wird die Verbindung der Formel XVII mit einem Dicarbonat der Formel XVIII umgesetzt.

Die Verbindungen der Formel XVII, Dicarbonate der Formel XVIII, Trihaloessigsäureester der Formel XIX, Azide der Formel XX, Carbonate der Formel XXI und Alkylideniminooxyameisensäureester der Formel XXII sind bekannte Substanzen. Sollten einige noch neu sein, so können sie in Analogie zu allgemein bekannten Methoden hergestellt werden.

Das jeweilige Molverhältnis zwischen der Verbindung der Formel XVII und den Verbindungen der Formeln XVIII - XXII richtet sich nach x, d.h. nach der Zahl der einzuführenden Resten B. Zweckmässig werden die Verbindungen der Formeln XVIII - XXII allerdings im 2-bis 10-fachem Ueberschuss eingesetzt.

Geeignete aprotische organische Lösungsmittel sind beispielsweise Ether, wie Tetrahydrofuran oder Dioxan, oder Glykoläther, wie Ethylenglykol-methyläther, Ethylenglykol-ethylether, Diethylenglykol-monomethylether oder Diethylenglykol-monoethylether, ferner dipolar-aprotische Lösungsmittel, wie Acetonitril, Benzonitril, N,N-Dimethylformamid, N,N-Dimethylacetamid, Nitrobenzol, N-Methylpyrrolidon, halogenierte aliphatische oder aromatische Kohlenwasserstoffe, wie Trichlorethan, Benzol oder durch Alkyl, Alkoxy oder Halogen substituiertes Benzol, wie Toluol, Xylol, Anisol oder Chlorbenzol oder aromatische N-Heterocyclen, wie Pyridin, Picolin oder Chinolin. Bevorzugte Lösungsmittel sind z.B. Tetrahydrofuran, N,N-Dimethylformamid, N-Methylpyrrolidon. Die genannten Lösungsmittel können auch als Mischungen eingesetzt werden. Zweckmässigerweise verwendet man 5-20 Gew.-Teile Lösungsmittel auf 1 Gew.-Teil der Reaktionsteilnehmer.

Als Katalysator geeignete Basen sind beispielsweise die Alkalimetalle selbst, wie Lithium-, Natrium- oder Kalium sowie deren Hydroxide und Carbonate, oder Alkaliamide, wie Lithium-, Natrium- oder Kaliumamid oder Alkalihydride, wie Lithium-, Natrium- oder Kaliumhydrid, oder Erdalkali- oder Alkalialkoholate, die sich insbesondere von primären, sekundären oder tertiären aliphatischen Alkoholen mit 1 bis 10 C-Atomen ableiten, wie z.B. Lithium-, Natrium- oder Kaliummethylat, -ethylat, -n-propylat, -isopropylat, n-butylat, -sek.-butylat, tert.-butylat, -2-methyl-2-butylat, -2-methyl-2-pentylat, -3-methyl-3-pentylat, -3-ethyl-3-pentylat, und ferner organische aliphatische, aromatische oder heterocyclische N-Basen, darunter z.B. Diazabicycloocten, Diazabicycloundecen und 4-Dimethylaminopyridin und Trialkylamine, wie z.B. Trimethyl- oder Triethylamin. Man kann aber auch ein Gemisch der genannten Basen verwenden.

Bevorzugt werden die organischen N-Basen, wie z.B. Diazabicyclooctan, Diazabicycloundecen und insbesondere 4-Dimethylaminopyridin.

Die Umsetzung wird bevorzugt bei Temperaturen zwischen 10 und 100°C, insbesondere zwischen 18 und 40°C, d.h. vorzugsweise bei Raumtemperatur, durchgeführt und zwar bei atmosphärischem Druck.

Für die Herstellung der erfindungsgemässen Verbindungen der Formel I geeignete Dicarbonate der Formel 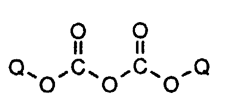 können auch nach einer neuen Methode, die Gegenstand einer Parallelanmeldung ist, durch Umsetzung mindestens eines Estercarbonates der Formel 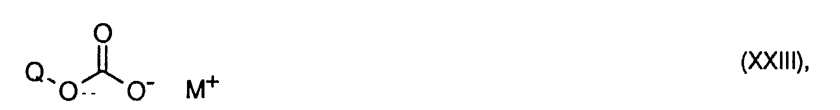
worin Q die oben angegebene Bedeutung hat, M⁺ für Na⁺, Li⁺, K⁺ oder NR₄₁R₄₂R₄₃R₄₄⁺ steht, und R₄₁ bis R₄₄ unabhängig voneinander Wasserstoff, C₁-C₁₈-Alkyl, C₅-C₁₀-Cycloalkyl oder C₇-C₁₈-Aralkyl bedeuten,
mit 40-50 Mol% eines Sulfochlorides der Formel 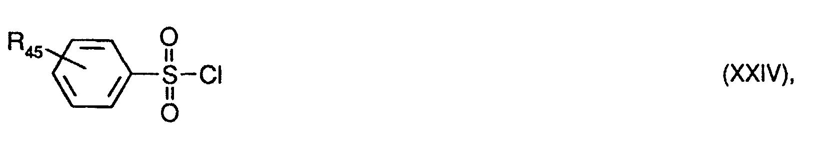
worin R₄₅ -H, -CH₃, -CH₂CH₃, -Cl, -Br, -OCH₃ oder -NO₂ bedeutet,
   in Gegenwart von 0.8-5 Mol% eines Katalysators der Formel 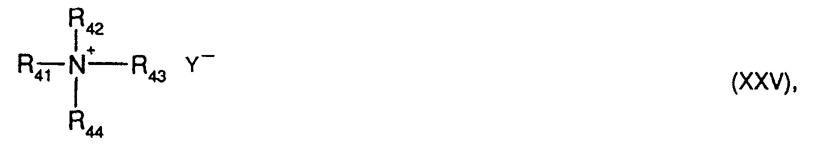
worin R₄₁ bis R₄₄ die oben angegebene Bedeutung haben, und
Y⁻ für ein nicht nukleophiles Anion steht,
und 1-5 Mol% eines heterozyklischen aromatischen Amins in einem unpolaren inerten Lösemittel,
wobei alle Molangaben auf 100 Mol % Estercarbonat der Formel XXIII bezogen sind, bei einer Temperatur zwischen -10°C und +25°C, hergestellt werden.

C₁-C₁₈-Alkyl für R₄₁-R₄₄ ist zum Beispiel Methyl, Ethyl, n-Propyl, Isopropyl, n-Butyl, sec.-Butyl, Isobutyl, tert.-Butyl, n-Pentyl, 2-Pentyl, 3-Pentyl, 2,2-Dimethylpropyl, n-Hexyl, n-Octyl, 1,1,3,3-Tetramethylbutyl, 2-Ethylhexyl, Nonyl, Decyl, Dodecyl, Tetradecyl, Hexadecyl oder Octadecyl.

C₄-C₁₂-Cycloalkyl ist zum Beispiel ein monozyklisches Cycloalkyl, wie beispielsweise Cyclobutyl, Cyclopentyl, Cyclohexyl, Trimethylcyclohexyl oder Menthyl, oder ein polyzyklisches Cycloalkyl, wie beispielsweise Thujyl, Bornyl, 1-Adamantyl oder 2-Adamantyl.

C₇-C₁₈-Aralkyl ist zum Beispiel 2-Benzyl-2-propyl, β-Phenyl-ethyl, α,α-Dimethylbenzyl, ω-Phenyl-butyl, ω-Phenyl-octyl, ω-Phenyl-dodecyl oder 3-Methyl-5-(1',1',3',3'-tetramethyl)butyl-benzyl. C₇-C₂₄-Aralkyl kann darüberhinaus beispielsweise auch 2,4,6-Tri-tert.-butyl-benzyl oder 1-(3,5-Dibenzyl-phenyl)-3-methyl-2-propyl bedeuten.

Heterozyklische aromatische Amine sind zum Beispiel Pyridin, α-, β- oder γ-Picolin, 2,4-, 2,6-, 3,4- oder 3,5-Lutidin, Collidin oder Chinolin.

Unpolare inerte Lösemittel sind solche, welche eine Dielektrizitätskonstante ε ≤ 10 haben, mit Wasser nicht mischbar sind und unter den Verfahrensbedingungen weder mit dem Estercarbonat der Formel (XXIII) noch mit dem Sulfochlorid der Formel (XXIV) reagieren, zum Beispiel aromatische Kohlenwasserstoffe, wie beispielsweise Benzol, Toluol, Xylol, Mesitylen oder Ethylbenzol, aliphatische Kohlenwasserstoffe, wie beispielsweise Pentan, Hexan, Cyclohexan, Heptan, Octan, Decan oder Dekalin, nicht-zyklische Ether, wie beispielsweise Diethylether, Diisopropylether oder Diisobutylether, oder Gemische davon, wie beispielsweise Siedegrenzebenzine oder ®Shell-Sol Produkte.

Bevorzugte Sulfochloride der Formel (XXIV) sind Benzolsulfochlorid und p-Toluolsulfochlorid.
Besonders bevorzugtes Sulfochlorid der Formel (XXIV) ist p-Toluolsulfochlorid.
Bevorzugte Katalysatorkationen sind solche, worin R₂ bis R₅ unabhängig voneinander Methyl, Ethyl, Butyl, Benzyl, Octyl, Dodecyl oder Octadecyl sind, insbesondere solche, worin die Summe der in den Gruppen R₄₁ bis R₄₄ enthaltenen Kohlenstoffatome eine Zahl von 10 bis 24 ist.
Besonders bevorzugte Katalysatorkationen sind solche, worin R₄₁ bis R₄₄ Butyl, oder R₄₁ und R₄₂ Methyl, R₄₃ Methyl oder Ethyl, und R₄₄ Benzyl, Dodecyl oder Octadecyl sind.
Nicht nukleophile Katalysatoranionen Y⁻ sind beispielsweise Cl⁻, Br⁻, F⁻, J⁻, NO₃-, ClO₄⁻, HSO₄⁻, PF₆⁻, B(C₆H₅)₄⁻ oder BF₄⁻.
Bevorzugte Katalysatoranionen sind Bromid und Chlorid, insbesondere Chlorid.
Besonders bevorzugter Katalysator der Formel (XXV) ist Benzyltrimethylammoniumchlorid.
Bevorzugtes heterozyklisches aromatisches Amin ist Pyridin.
Bevorzugte Lösemittel sind aromatische Kohlenwasserstoffe.
Besonders bevorzugte Lösemittel sind Toluol und Xylol.
Die bevorzugte Menge Sulfochlorid der Formel (XXIV) beträgt zirka 45 Mol%, bezogen auf (XXIII).
Die bevorzugte Menge Katalysator der Formel (XXV) beträgt 1.0-1.5 Mol%, bezogen auf (XXIII).
Die bevorzugte Menge heterozyklisches aromatisches Amin beträgt 1-3 Mol%, bezogen auf (XXIII). Die besonders bevorzugte Menge heterozyklisches aromatisches Amin beträgt zirka 3 Mol%, bezogen auf (XXIII).
Die Menge Lösemittel ist nicht kritisch. Bevorzugt wird gerade so viel Lösemittel verwendet, dass das Reaktionsgemisch während der ganzen Reaktion gut rührbar bleibt; je nach herzustellendem Pyrokohlensäurediester kann diese Menge verschieden sein.
Die bevorzugte Reaktionstemperatur beträgt zwischen 0°C und +20°C.
Die besonders bevorzugte Reaktionstemperatur beträgt zwischen 0°C und +10°C.

Die Reaktionszeit hängt von den Mengen Katalysator und heterozyklisches aromatisches Amin sowie von der Temperatur ab. Üblicherweise ist die Umsetzung nach ½ bis 100 Stunden, bevorzugt nach ½ bis 10 Stunden beendet.

Alle benötigten Chemikalien sind bekannt. Sie sind kommerziell erhältlich oder können nach bekannten Methoden hergestellt werden.

Das Verfahren kann in einfachster Weise durchgeführt werden, indem das Lösemittel und alle Edukte (XXIII) bis (XXV) gleichzeitig oder nacheinander in beliebiger Reihenfolge bei der Reaktionstemperatur ins Reaktionsgefäss eingeführt werden, wobei zweckmässig mindestens ein Teil des Lösemittels vorgelegt, und das Sulfochlorid zuletzt zugegeben werden.

Die erfindungsgemässen Verbindungen der Formel I eignen sich ausgezeichnet als Fluoreszenzfarbstoffe zum Färben von hochmolekularem organischem Material in der Masse.

Beispiele geeigneter hochmolekularer organischer Materialien, die mit den erfindungsgemässen Verbindungen der Formel I gefärbt werden können, sind Vinylpolymere, wie z.B. Polystyrol, Poly-α-methylstyrol, Poly-p-methylstyrol, Poly-p-hydroxystyrol, Poly-p-hydroxyphenylstyrol, Poly(methylacrylat) und Poly(acrylamid) sowie die entsprechenden Methacrylverbindungen, Poly(methylmaleat), Poly(acrylnitril), Poly(methacrylnitril), Poly(vinylchlorid), Poly(vinylfluorid), Poly(vinylidenchlorid), Poly(vinylidenfluorid), Poly(vinylacetat), Poly(methylvinylether) und Poly(butylvinylether); Novolake abgeleitet von C₁-C₆-Aldehyden, wie z.B. Formaldehyd und Acetaldehyd, und einem zweikernigen, vorzugsweise einkernigen Phenol, das gegebenenfalls mit einer oder zwei C₁-C₉-Alkylgruppen, einem oder zwei Halogenatomen oder einem Phenylring substituiert ist, wie beispielsweise o-, m- oder p-Kresol, Xylol, p-tert-Butylphenol, o-, m- oder p-Nonylphenol, p-Chlorphenol oder p-Phenylphenol, oder einer Verbindung mit mehr als einer phenolischen Gruppe, wie z.B. Resorcin, Bis-(4-hydroxyphenyl)methan oder 2,2-Bis-(4-hydroxyphenyl)propan; von Maleinimid und/oder Maleinanhydrid abgeleitete Polymere, wie z.B. Copolymere von Maleinanhydrid und Styrol; Poly(vinylpyrrolidon), Biopolymere und deren Derivate, wie z.B. Cellulose, Stärke, Chitin, Chitosan, Gelatine, Zein, Ethylcellulose, Nitrocellulose, Celluloseacetat und Cellulosebutyrat; natürliche Harze und Kunstharze, wie z.B. Gummi, Casein, Silikon und Silikonharze, ABS, Harnstoff- und Melamin-Formaldehydharze, Alkydharze, Phenolharze, Polyamide, Polyimide, Polyamid/imide, Polysulfone, Polyethersulfone, Polyphenylenoxide, Polyurethane, Polyharnstoffe, Polycarbonate, Polyarylene, Polyarylensulfide, Polyepoxide, Polyolefine und Polyalkadiene. Bevorzugte hochmolekulare organische Materialien, sind z.B. Celluloseether und -ester, wie Ethylcellulose, Nitrocellulose, Celluloseacetat oder Cellulosebutyrat, natürliche Harze oder Kunstharze, wie Polymerisations- oder Kondensationsharze, wie Aminoplaste, insbesondere Harnstoff- und Melamin-Formaldehydharze, Alkydharze, Phenoplaste, Polycarbonate, Polyolefine, Polystyrol, Polyvinylchlorid, Polyamide, Polyurethane, Polyester, ABS, Polyphenylenoxide, Gummi, Casein, Silikon und Silikonharze, einzeln oder in Mischungen.

Die erwähnten hochmolekularen organischen Verbindungen können einzeln oder in Gemischen als plastische Massen, Schmelzen oder in Form von Spinnlösungen, Lacken, Anstrichstoffen oder Druckfarben vorliegen. Je nach Verwendungszweck erweist es sich als vorteilhaft, die erfindungsgemässen Verbindungen der Formel I als Toner oder in Form eines Präparates einzusetzen.

Besonders geeignet sind die erfindungsgemässen Verbindungen der Formel I zum Massefärben von Polyvinylchlorid und insbesondere Polyolefinen, wie Polyethylen und Polypropylen, sowie von Lacken, auch von Pulverlacken, Druckfarben und Anstrichstoffen.

Bezogen auf das zu färbende hochmolekulare organische Material kann man die erfindungsgemässen Verbindungen der Formel I in einer Menge von 0,01 bis 30 Gew.%, vorzugsweise von 0,1 bis 10 Gew.%, einsetzen.

Die Einfärbung der hochmolekularen organischen Substanzen mit den erfindungsgemässen Verbindungen der Formel I erfolgt beispielsweise derart, dass man die Verbindung der Formel I, gegebenenfalls in Form von Masterbatches, diesen Substraten unter Verwendung von Walzwerken, Misch- oder Mahlapparaten zumischt. Das gefärbte Material wird hierauf nach an sich bekannten Verfahren, wie Kalandrieren, Pressen, Strangpressen, Streichen, Giessen oder Spritzgiessen, in die gewünschte endgültige Form gebracht. Oft ist es erwünscht, zur Herstellung von nicht starren Formlingen oder zur Verringerung ihrer Sprödigkeit den hochmolekularen Verbindungen vor der Verformung sogenannte Weichmacher einzuverleiben. Als solche können z.B. Ester der Phosphorsäure, Phthalsäure oder Sebacinsäure dienen. Die Weichmacher können vor oder nach der Einverleibung der erfindungsgemässen Verbindungen der Formel I in die Polymeren eingearbeitet werden. Zwecks Erzielung verschiedener Farbtöne ist es ferner möglich, den hochmolekularen organischen Stoffen neben der erfindungsgemässen Verbindung noch Füllstoffe bzw. andere farbgebende Bestandteile, wie Weiss-, Bunt- oder Schwarzpigmente, in beliebigen Mengen zuzufügen.

Zum Einfärben von Lacken, Anstrichstoffen und Druckfarben werden die hochmolekularen organischen Materialien und die erfindungsgemässen Verbindungen der Formel I gegebenenfalls zusammen mit Zusatzstoffen, wie Füllmitteln, Pigmenten, Siccativen oder Weichmachern, in einem gemeinsamen organischen Lösungsmittel oder Lösungsmittelgemisch fein dispergiert bzw. gelöst. Man kann dabei so verfahren, dass man die einzelnen Komponenten für sich oder auch mehrere gemeinsam dispergiert bzw. löst, und erst hierauf alle Komponenten zusammenbringt.

In Färbungen, beispielsweise von Polyvinylchlorid oder Polyolefinen oder in Druckfarben, zeichnen sich die erfindungsgemässen Verbindungen der Formel I durch gute allgemeine Eigenschaften, wie gute Migrations-, Licht- und Wetterbeständigkeit, aber insbesondere durch die unerwartet hohe Fluoreszenz aus.

Von ganz grosser Bedeutung ist aber die ganz unerwartete Leichtigkeit mit der die erfindungsgemässen löslichen Chromophore, selbst im Substrat in welchem sie bereits eingearbeitet wurden, zu den entsprechenden Pigmenten der Formel A(H)ₓ umgewandelt werden können. Dies kann auf einfachste Weise erzielt werden, durch thermische Behandlung (Aufheizen auf Temperaturen zwischen 50 und 210°C, bevorzugt zwischen 100 und 170°C, je nach Pigment, der die erfindungsgemässen löslichen Verbindungen enthaltenden Festkörper, Lösungen oder Dispersionen in organischen oder wässrigen Medien, Polymerlösungen oder Schmelzen. Dies erlaubt die Einfärbung von Lacken, Druckfarben, insbesondere für Inkjet und Kunststoffen, auch z.B. in Faserform, mit insgesamt verbesserten Eigenschaften, wie Reinheit, Farbstärke, Brillianz und Transparenz, sowie interessante Applikationen in der Analytik.

Ein weiterer Gegenstand der Erfindung betrifft demnach hochmolekulares organisches Material enthaltend in der Masse ein in situ durch thermischen Abbau einer löslichen Verbindung der Formel I erzeugtes Pigment der Formel A(H)ₓ, sowie thermo-, photo- oder chemosensitives Aufzeichnungsmaterial und auch photo- und elektrolumineszente Materialien enthaltend eine erfindungsgemässe Verbindung der Formel I.

Es ist sogar gefunden worden, dass im Fall bestimmter Verbindungen der Formel I die thermische Behandlung durch Erhitzen auf Temperaturen von 100-180°C, bevorzugt 105-120°C zu Kristallmodifikationsumwandlungen der entsprechenden Chromophore XVII führen kann.

Ein zusätzlicher Gegenstand der Erfindung ist daher auch ein Verfahren zur Kristallmodifikationsumwandlung von Chromophoren der Formel XVII durch Umsetzung in Verbindungen der Formel I z.B. nach dem obenerwähnten Verfahren, und thermische Behandlung bei Temperaturen zwischen 100 und 180°C der erhaltenen Verbindungen der Formel I.

Die nachfolgenden Beispiele erläutern die Erfindung.

### A) Herstellung der Dicarbonate

Beispiel A1: Einer auf 3°C gekühlten Lösung von 84,1 g (1 mol) 2-Methyl-3-butin-2-ol in 1,75 1 Toluol werden unter Schutzgasatmosphäre 40 g (1 mol) 60 %iges Natriumhydrid portionenweise so zugegeben, dass die Temperatur 10°C nicht übersteigt. Nach Zugabe von 250 ml Toluol wird das Gemisch über Nacht bei 18°C gerührt. Nach Abkühlung der braunen Lösung auf 5°C werden 101,7 g (2,3 mol) CO₂ bei 5-10°C eingeleitet. Zum auf 18°C erwärmten Reaktionsgemisch werden nacheinander 3,2 g (0,014 mol) Benzyltrimethylammoniumchlorid, 2,4 g (0,03 mol) Pyridin und 82,8 g (0,43 mol) Toluol-4-sulfochlorid gegeben. Die entstehende Suspension wird drei Tage bei Raumtemperatur weitergerührt. Bei 5°C werden dann 260 ml 5%ige wässerige H₂SO₄ so zugetropft, dass die Temperatur 10°C nicht übersteigt. Die organische Phase wird abgetrennt, fünfmal mit je 400 ml Wasser gewaschen, über Na₂SO₄ getrocknet und im Vakuum eingeengt. Das Rohprodukt (94,8 g) wird mit Hexan behandelt, wobei man 70 g (59% d.Th., berechnet auf das eingesetzte Toluol-4-sulfochlorid) reines weisses Di-(2-methyl-3-butin-2-yl)-dicarbonat vom Schmelzpunkt 102,8°C erhält.

| Analyse: | C | H |
|---|---|---|
| Berechnet | 60,50 % | 5,92 % |
| Gefunden | 60,41 % | 6,16% |

Beispiel A2: Beispiel A1 wird wiederholt, jedoch ausgehend von 2-Methyl-3-buten-2-ol anstatt 2-Methyl-3-butin-2-ol, und mit 0,50 mol Toluol-4-sulfochlorid anstatt 0,40 mol Toluol-4-sulfochlorid. Man erhält Di-(2-methyl-3-buten-2-yl)-dicarbonat in guter Qualität mit einer Ausbeute von 61% d.Th. (berechnet auf das eingesetzte Toluol-4-sulfochlorid).
Analyse:
¹H-NMR (CDCl₃: 6,08 (dd 2H); 5,29 (d, 2H); 5,19 (d, 2H); 1,60 (s, 12H)
Beispiel A3: Beispiel A1 wird wiederholt, jedoch ausgehend von 3-Methyl-2-buten-1-ol anstatt 2-Methyl-3-butin-2-ol. Man erhält Di-(3-methyl-2-buten-1-yl)-dicarbonat mit einem ¹H-NMR-Spektrum in gutem Einklang mit der Erwartung.
Analyse:
¹H-NMR (CDCl₃): 5,38 (m, 2H); 4,73 (d, 4H); 1,77 (s, -CH₃); 1,73 (s, -CH₃)

### B) Herstellung der erfindungsgemässen Verbindungen

Beispiel B1 (nicht erfindungsgemäß): Einer Suspension von 2 g (6,94 x 10⁻³ Mol) Pyrrolo[3,4-c]pyrrol de Formel 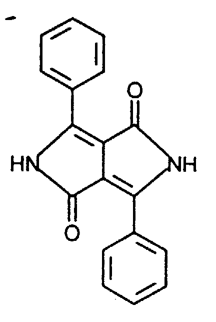 in 50 ml Tetrahydrofuran werden 0,085 g (6,94 x 10⁻⁴ Mol) Dimethylaminopyridin und einen grossen Ueberschuss an Di-(2-methyl-3-buten-2-yl)-dicarbonat aus Beispiel A2 (10 g; 4,1 x 10⁻² Mol) zugegeben. Es wird über Nacht bei Zimmertemperatur gerührt und danach wird das Lösungsmittel aus der fluoreszenten orange-braunen Lösung abdestilliert. Durch Zugabe von Hexan zum Rückstand fällt eine orange feste Substanz aus, die abfiltriert und im Vakuumtrockenschrank getrocknet wird. Man erhält somit 2,39 g (67 % d.Th.) der Verbindung der Formel

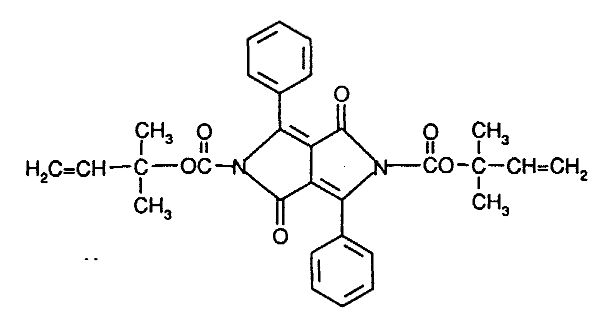

| Analyse: | C | H | N |
|---|---|---|---|
| Berechnet | 70,30 % | 5,51 % | 5,47 % |
| Gefunden | 70,30 % | 5,57 % | 5,38 % |

Beispiel B2: Beispiel B1 wird wiederholt, mit der Ausnahme, dass anstelle von Di-(2-methyl-3-buten-2-yl)-dicarbonat die äquivalente Menge Di-(2-methyl-3-butin-2-yl)-dicarbonat aus Beispiel A1 verwendet wird. Man erhält somit die Verbindung der Formel 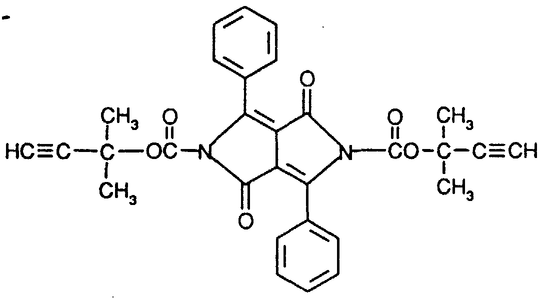 Analyse: ¹H-NMR (CD₂Cl₂): 7,75 (m, 4H); 7,50 (m, 6H); 2,71 (s, 2H); 1,52 (s, 12H)
Beispiel B3: Beispiel B1 wird wiederholt, mit der Ausnahme, dass anstelle von Di-(2-methyl-3-buten-2-yl)-dicarbonat die äquivalente Menge Di-(3-methyl-2-buten-1-yl)-dicarbonat aus Beispiel A3 verwendet wird. Man erhält somit die Verbindung der Formel 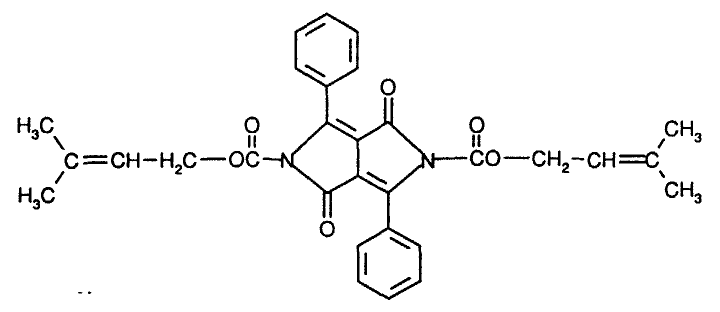 Analyse:
¹H-NMR (CD₂Cl₂): 7,71 (m, 4H); 7.48 (m, 6H); 5,20 (t, 2H); 4,70 (d, 4H); 1,73 (s, 6H); 1,63 (s, 6H)
Beispiel B4: Beispiel B1 wird wiederholt, mit der Ausnahme, dass anstelle des Pyrrolo[3,4-c]pyrrols die äquivalente Menge des Chinacridons der Formel 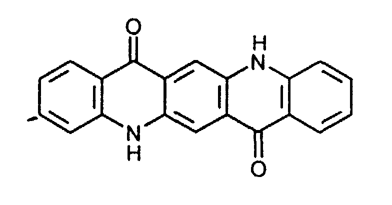 verwendet wird. Man erhält somit die Verbindung der Formel 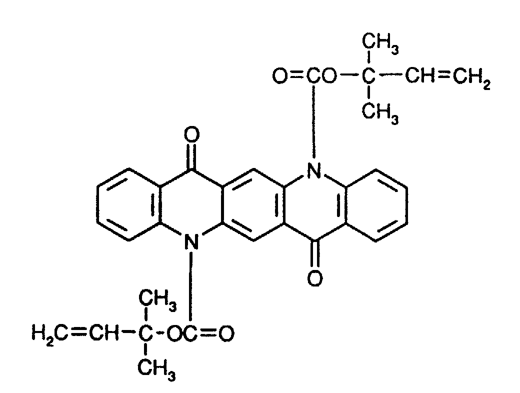 Analyse:
¹H-NMR (CD₂Cl₂): 8,75 (m, 2H); 8,35 (m, 2H); 7,86 (m, 2H); 7,72 (m, 2H); 7,40 (m, 2H); 6,36 (dd, 2H); 5,43 (d, 2H); 5,32 (d, 2H); 1,77 (s, 6H); 1,76 (s, 6H)
Beispiel B5: Beispiel B4 wird wiederholt, mit der Ausnahme, dass anstelle von Di-(2-methyl-3-buten-2-yl)-dicarbonat die äquivalente Menge Di-(2-methyl-3-butin-2-yl)-dicarbonat verwendet wird. Man erhält somit die Verbindung der Formel 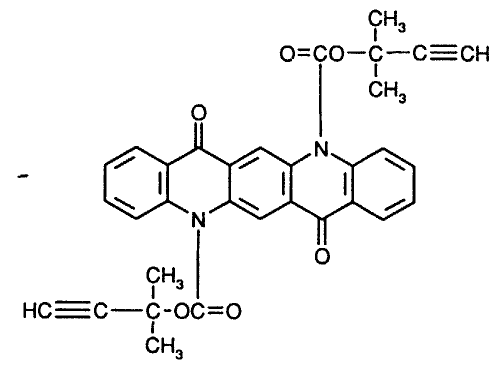 Analyse:
¹NMR (CD₂Cl₂): 8,78 (s, 2H); 8,33 (d, 2H); 7,93 (d, 2H); 7,74 (t, 2H); 7,42 (t, 2H); 2,89 (s, 2H); 1,87 (s, 12H)
Beispiel B6: Einer Suspension von 1 g (3,8 x 10⁻³ Mol) Indigo in 40 ml Tetrahydrofuran werden 0,1 g (8,2 x 10⁻⁴ Mol) Dimethylaminopyridin und 1,85 g (7,6 x 10⁻³ Mol) Di-(2-methyl-3-buten-2-yl)-dicarbonat zugegeben. Es wird über Nacht unter Argon bei Zimmertemperatur gerührt, die Lösung danach filtriert und das Filtrat eingeengt. Durch Zugabe von Pentan zum Rückstand fällt eine violette feste Substanz aus, die abfiltriert und im Vakuumtrockenschrank getrocknet wird. Man erhält somit 0,77 g (42 % d.Th.) der Verbindung der Formel

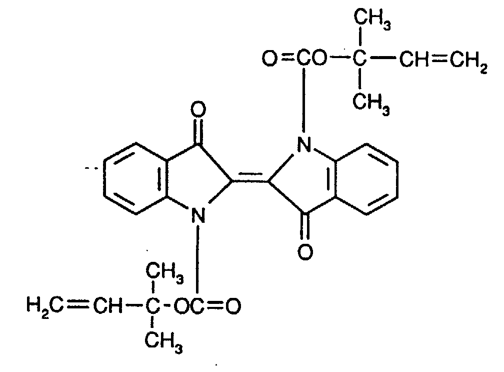

| Analyse: | C | H | N |
|---|---|---|---|
| Berechnet | 69,12 % | 5.39 % | 5.76 % |
| Gefunden | 68,65 % | 5,64 % | 5,68 % |

### C) Applikationsbeispiele

Durch Erhitzen der in der ersten Kolonne der nachfolgenden Tabelle angegebenen Produkte auf die unter T angegebene Mittelpunkttemperatur, bei einer Aufheizrate von 10°C/Min, werden die entsprechenden Pigmente (Produkte der angegebenen Formeln, worin Q' Wasserstoff bedeutet) zurückgebildet.
a) Chinacridone 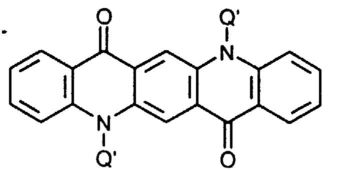 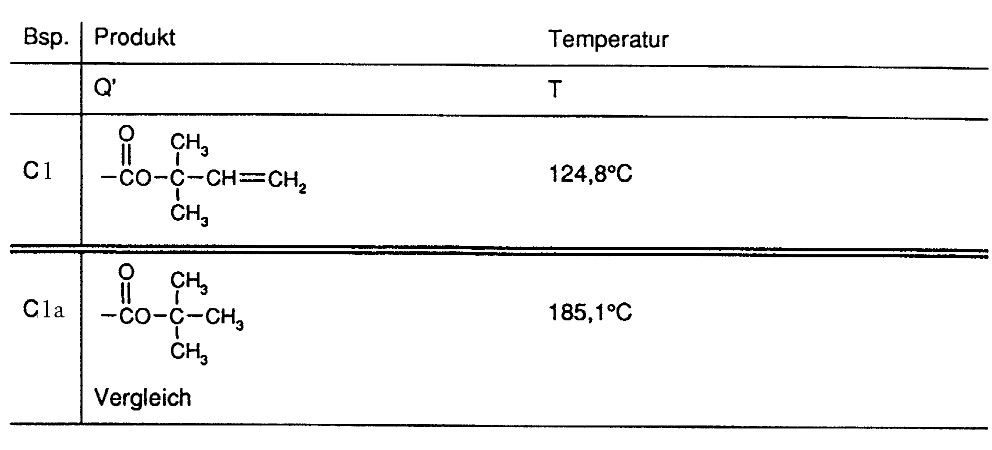
C) Indigo

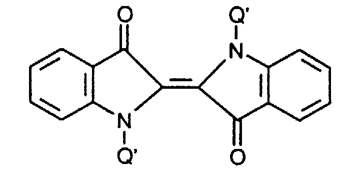

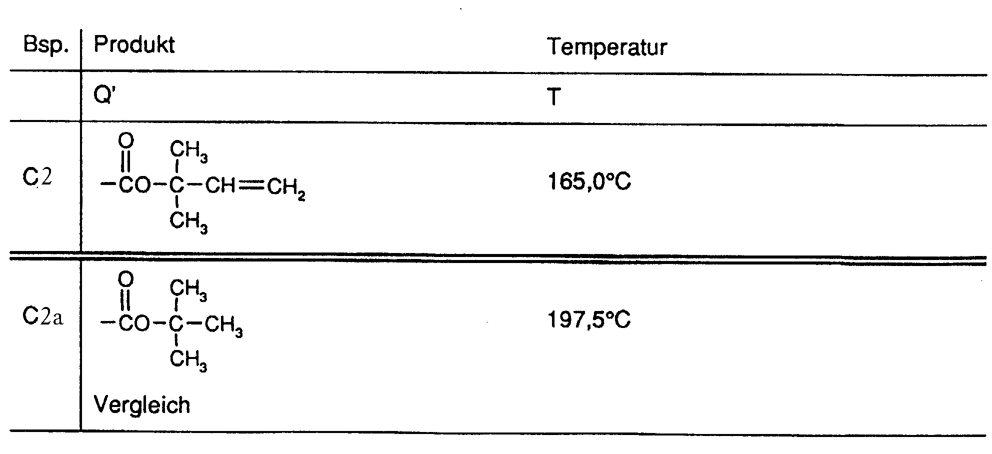

## Patentansprüche

1. Verbindungen der Formel
A(B)ₓ (I),
worin x eine ganze Zahl zwischen 1 und 4 bedeutet,
A für den Rest eines Chromophors der Chinacridon-, Anthrachinon-, Perylen-, Indigo-, Chinophthalon-, Isoindolinon-, Isoindolin-, Dioxazin, Phthalocyanin, Diketopyrrolopyrrol- oder Azoreihe steht, der x mit B verbundene N-Atome, vorzugsweise mit mindestens einer unmittelbar benachbarten oder konjugierten Carbonylgruppe, enthält.
B für eine Gruppe der Formel 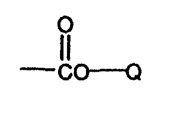 steht, und, wenn x 2, 3 oder 4 bedeutet, auch ein-, zwei- oder dreimal Wasserstoff sein kann, wobei Q eine Gruppe der Formel 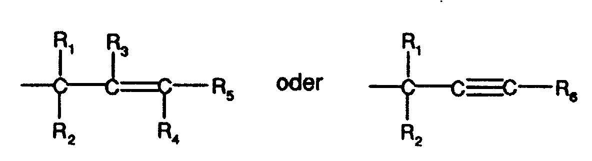 ist, worin
R₁ und R₂ unabhängig voneinander Wasserstoff, C₁-C₂₄- Alkyl, durch O, S oder N(R₉)₂ unterbrochenes C₁-C₂₄-Alkyl, C₃-C₂₄-Alkenyl, C₃-C₂₄-Alkinil, C₄-C₁₂-Cycloalkyl, C₄-C₁₂-Cycloalkenyl, unsubstituiertes oder durch C₁-C₆-Alkyl, C₁-C₆-Alkoxy, Halogen, Cyano oder Nitro substituiertes Phenyl oder Biphenyl bedeuten, R₃, R₄ und R₅ unabhängig voneinander Wasserstoff, C₁-C₂₄-Alkyl oder C₃-C₂₄-Alkenyl sind,
R₆ Wasserstoff, C₁-C₂₄-Alkyl, C₃-C₂₄-Alkenyl oder eine Gruppe der Formel 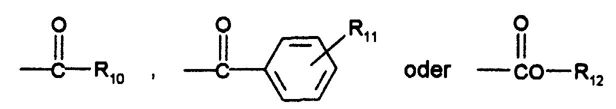 ist,
R₉ und R₁₀ C₁-C₆-Alkyl, R₁₁ Wasserstoff oder C₁-C₆-Alkyl und R₁₂ Wasserstoff, C₁-C₆-Alkyl, unsubstituiertes oder durch C₁-C₆-Alkyl substituiertes Phenyl sind,
mit der Massgabe, dass wenn A für den Rest eines Chromophors der Diketopyrrolopyrrolreihe steht, Q keine Gruppe der Formel 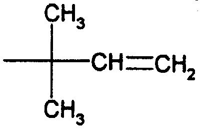 ist.

2. Verbindungen gemäss Anspruch 1 der Formel I, worin R₁ C₁-C₂₄-Alkyl, C₃-C₂₄-Alkenyl, C₃-C₂₄-Alkinyl, unsubstituiertes oder durch C₁-C₆-Alkyl, C₁-C₆-Alkoxy, Halogen, Cyano oder Nitro substituiertes Phenyl und R₆ C₁-C₂₄-Alkyl oder C₃-C₂₄-Alkenyl bedeuten.

3. Verbindungen gemäss Anspruch 1, der Formel I, worin
R₁ und R₂ Methyl sind,
R₃, R₄ und R₅ unabhängig voneinander Wasserstoff oder C₁-C₁₂-Alkyl bedeuten,
R₆ C₁-C₆-Alkyl ist und

4. Verbindungen gemäss Anspruch 3, der Formel I, worin
B eine Gruppe der Formel 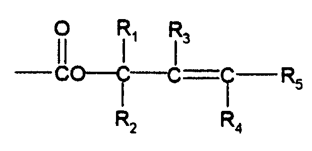 ist,
R₁ und R₂ Methyl und R₃, R₄ und R₅ unabhängig voneinander Wasserstoff oder C₁-C₁₂-Alkyl bedeuten.

5. Verbindungen gemäss Anspruch 1 ausgewählt aus der Gruppe bestehend aus
a) Perylencarbonsäureimiden der Formel 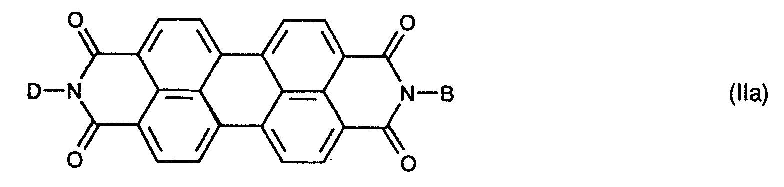 oder 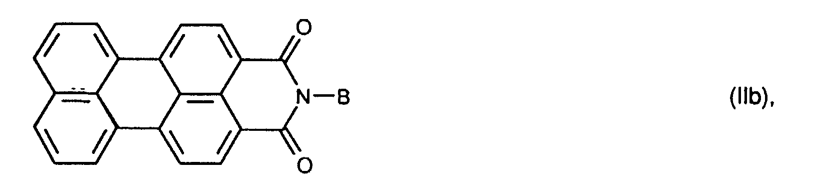 worin D Wasserstoff, C₁-C₆-Alkyl, unsubstituiertes oder durch Halogen oder C₁-C₆-Alkyl substituiertes Phenyl, Benzyl oder Phenethyl oder B bedeutet,
b) Chinacridonen der Formel 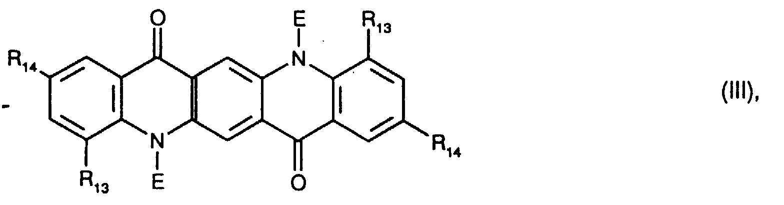 worin R₁₃ und R₁₄ unabhängig voneinander Wasserstoff, Halogen, C₁-C₂₄-Alkyl, C₁-C₆-Alkoxy oder Phenyl ist,
c) Dioxazinen der Formel 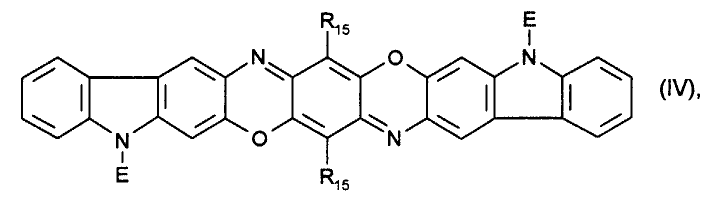 worin R₁₅ Wasserstoff, Halogen oder C₁-C₂₄-Alkyl ist,
oder der Formel 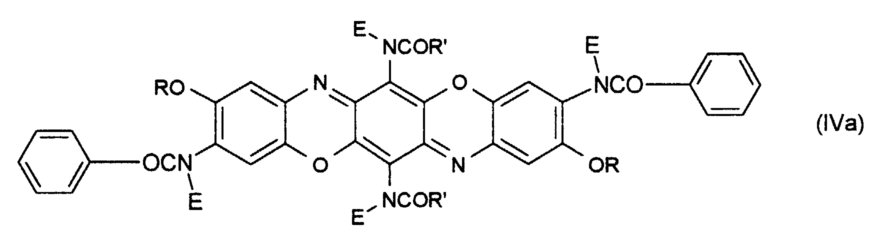 worin R und R' unabhängig voneinander C₁-C₄-Alkyl sind,
d) Isoindolinen der Formeln 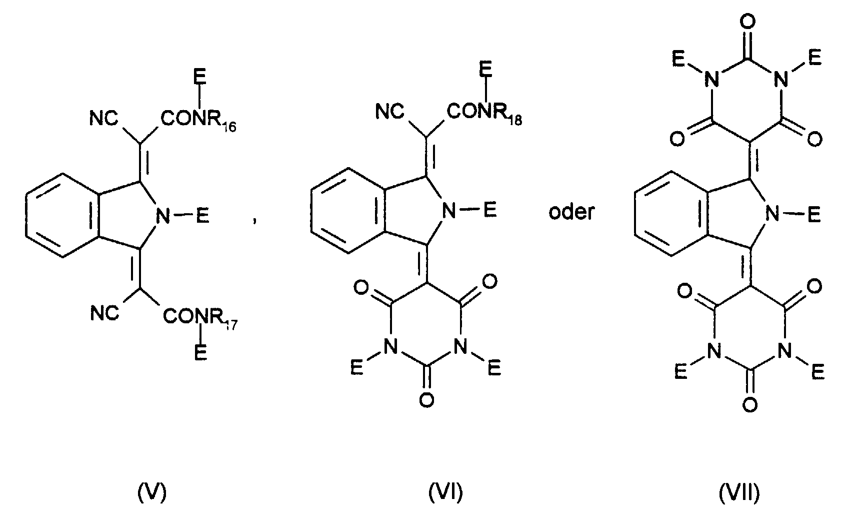 worin R₁₆ eine Gruppe 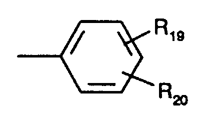
R₁₇ Wasserstoff, C₁-C₂₄-Aklyl, Benzyl oder eine Gruppe 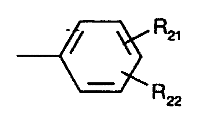 bedeutet
R₁₈ die gleiche Bedeutung wie R₁₆ hat,
R₁₉, R₂₀, R₂₁ und R₂₂ unabhängig voneinander Wasserstoff, C₁-C₂₄-Alkyl, C₁-C₆-Alkoxy, Halogen oder Trifluormethyl bedeuten,
e) Indigoderivate der Formel 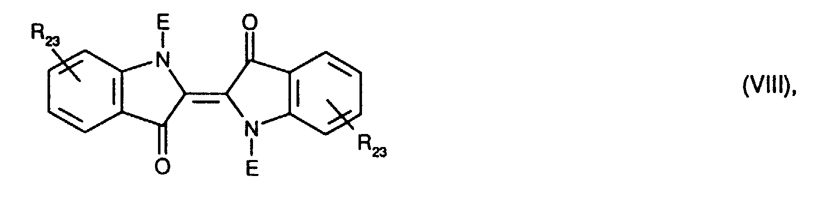 worin R₂₃ Wasserstoff, CN, C₁-C₆-Alkyl, C₁-C₆-Alkoxy oder Halogen bedeutet,
f) Benzimidazolon-Azoverbindungen der Formel 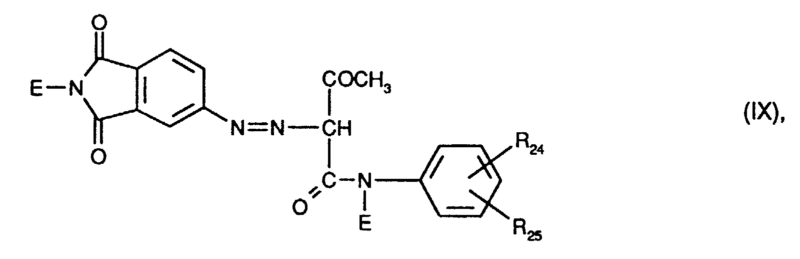 worin R₂₄ und R₂₅ unabhängig voneinander Wasserstoff, Halogen, C₁-C₆-Alkyl oder C₁-C₆-Alkoxy sind,
g) anthrachinoiden Verbindungen der Formel 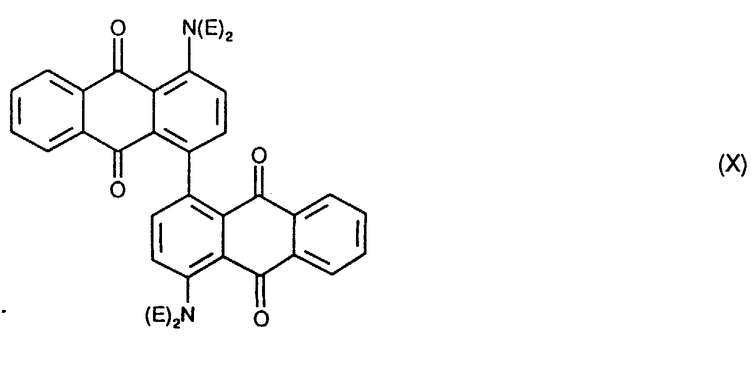 oder 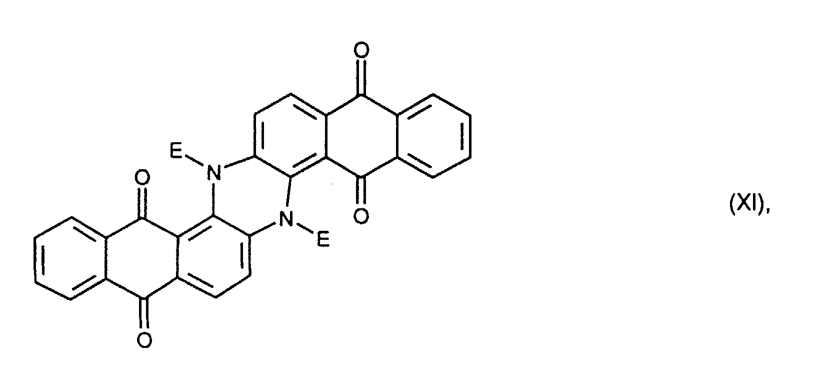
h) Phthalocyaninen der Formel 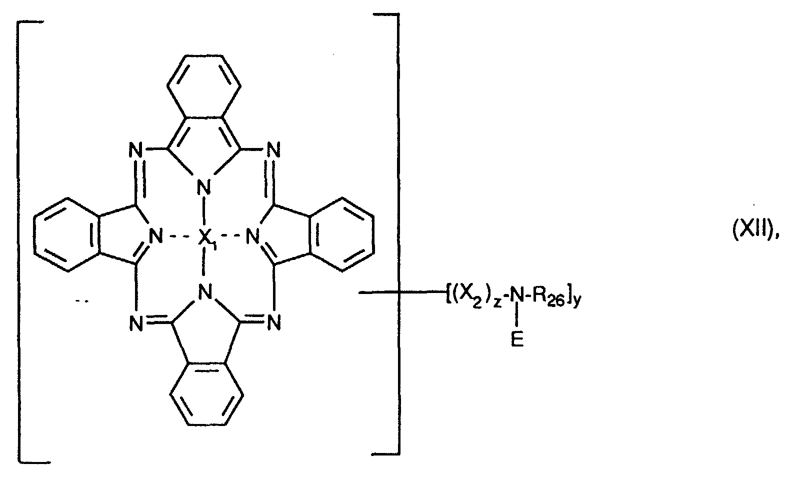 worin
X₁ H₂, ein zweiwertiges Metall ausgewählt aus der Gruppe Cu(II), Zn(II), Fe(II), Ni(II), Ru(II), Rh(II), Pd(II), Pt(II), Mn(II), Mg(II), Be(II), Ca(II), Ba(II), Cd(II), Hg(II), Sn(II), Co(II) oder Pb(II) oder ein zweiwertiges Oxometall ausgewählt aus der Gruppe V(O), Mn(O) oder TiO,
X₂-CH(R₂₄)-, -CO- oder -SO₂-
R₂₆ Wasserstoff, C₁-C₆-Alkyl, -N(E)R₂₇, -NHCOR₂₈₋ -COR₂₈ oder 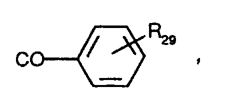
R₂₇ Wasserstoff oder C₁-C₆-Alkyl, R₂₈ C₁-C₆-Alkyl und R₂₉ Wasserstoff, Halogen, C₁-C₆-Alkyl oder C₁-C₆-Alkoxy bedeuten,
z für Null oder 1 und y für eine Zahl von 1 bis 4 stehen,
i) Isoindolinone der Formel 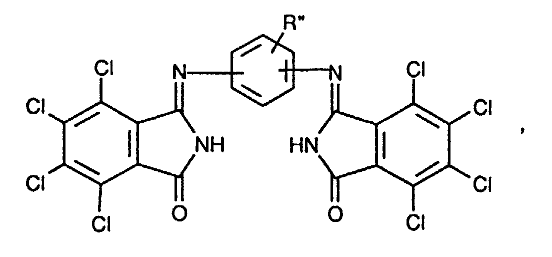 worin R" Wasserstoff oder C₁-C₄-Alkyl ist,
und
j) Pyrrolo[3,4-c]pyrrolen der Formel 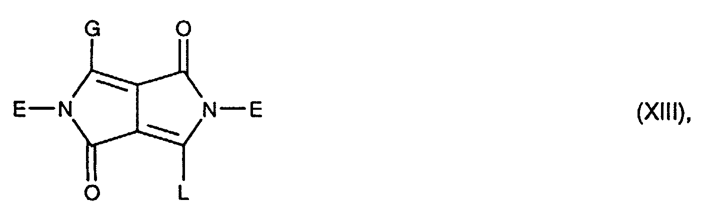 worin G und L unabhängig voneinander für eine Gruppe der Formel 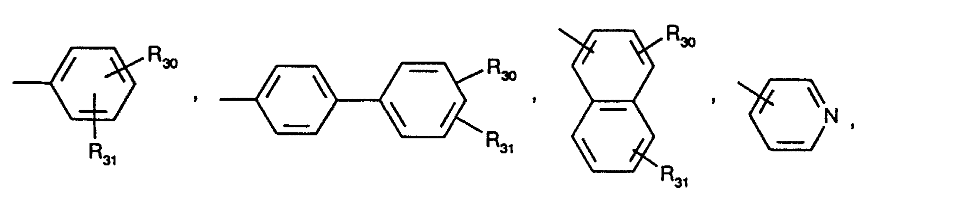 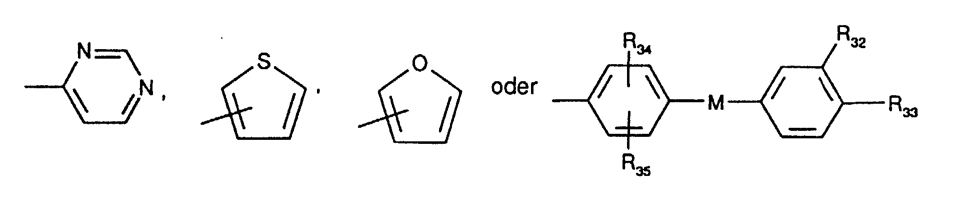 stehen, worin
R₃₀ und R₃₁ unabhängig voneinander Wasserstoff, Halogen, C₁-C₂₄-Alkyl, C₁-C₆-Alkoxy, C₁-C₁₈-Alkylmercapto, C₁-C₁₈-Alkylamino, -CN, -NO₂, -Phenyl, Trifluormethyl, C₅-C₆-Cycloalkyl, -C=N-(C₁-C₂₄-Alkyl), 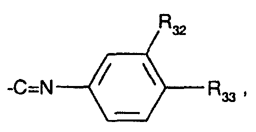 Imidazolyl, Pyrrazolyl, Triazolyl, Piperazinyl, Pyrrolyl, Oxazolyl, Benzoxazolyl, Benzthiazolyl, Benzimidazolyl, Morpholinyl, Piperidinyl oder Pyrrolidinyl bedeuten,
M -CH₂-, -CH(CH₃)-, -C(CH₃)₂-, -CH=N-, -N=N-, -O-, -S-, -SO-, -SO₂- oder -NR₃₆- ist,
R₃₂ und R₃₃ unabhängig voneinander Wasserstoff, Halogen, C₁-C₆-Alkyl, C₁-C₆-Alkoxy oder -CN sind, R₃₄ und R₃₅ unabhängig voneinander Wasserstoff, Halogen oder C₁-C₆-Alkyl und
R₃₆ Wasserstoff oder C₁-C₆-Alkyl bedeuten,
wobei in den obenerwähnten Formeln E jeweils Wasserstoff oder B bedeutet, mit der Massgabe, dass E in jeder Formel mindestens einmal B ist, und B die in Anspruch 1 angegebene Bedeutung hat.

6. Verbindungen gemäss Anspruch 5 ausgewählt aus der Gruppe bestehend aus
Chinacridonen der Formel III, worin R₁₃ und R₁₄ unabhängig voneinander Wasserstoff, Chlor oder Methyl bedeuten, oder
Pyrrolo[3,4-c]pyrrolen der Formel XIII, worin G und L gleich sind und eine Gruppe der Formel 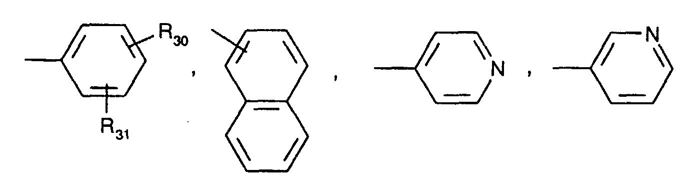 oder 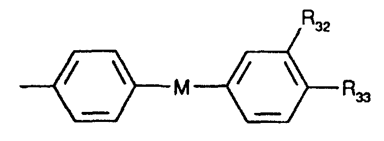 sind,
worin R₃₀ und R₃₁ unabhängig voneinander Wasserstoff, Chlor, Brom, C₁-C₄-Alkyl, C₁-C₆-Alkoxy, C₁-C₆-Alkylamino, CN oder Phenyl bedeuten,
M -O-, -NR₃₆-, -N=N- oder -SO₂- ist,
R₃₂ und R₃₃ Wasserstoff und R₃₆ Wasserstoff, Methyl oder Ethyl bedeuten, oder
anthrachinoiden Verbindungen der Formel XI,
wobei E in den Formeln III, XIII und XI jeweils die in Anspruch 5 angegebene Bedeutung hat.

7. Verbindungen gemäss Anspruch 4 der Formeln 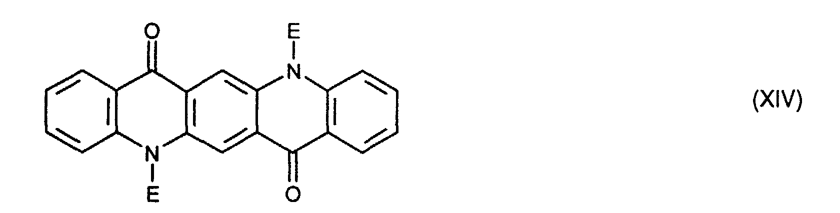 oder 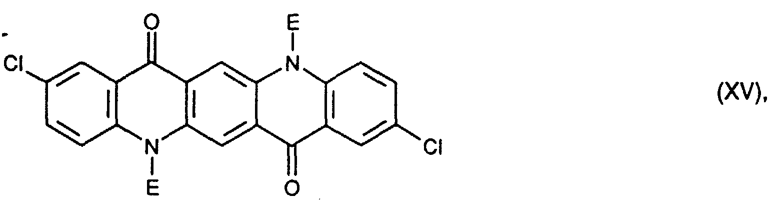 worin E Wasserstoff oder B bedeutet, mit der Massgabe, dass E in jeder Formel mindestens einmal B ist, und B die in Anspruch 4 angegebene Bedeutung hat.

8. Verbindungen gemäss Anspruch 4 der Formel 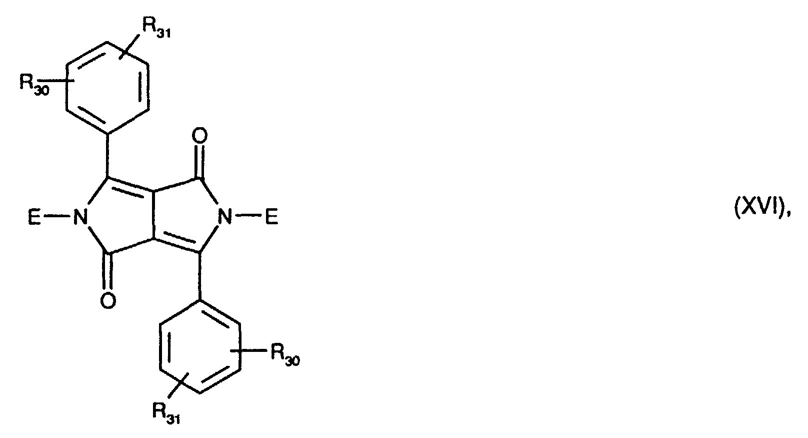 worin R₃₀ und R₃₁ unabhängig voneinander Wasserstoff, Methyl, tert.-Butyl, Chlor, Brom, CN oder Phenyl bedeuten, und
E Wasserstoff oder B bedeutet, mit der Massgabe, dass E mindestens einmal B ist, und B die in Anspruch 4 angegebene Bedeutung hat.

9. Verbindungen gemäss Anspruch 4 der Formel 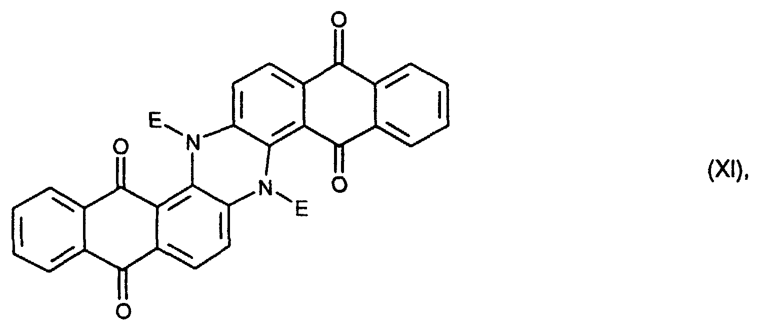 worin E Wasserstoff oder B bedeutet, mit der Massgabe, dass E mindestens einmal B ist, und B die in Anspruch 4 angegebene Bedeutung hat.

10. Hochmolekulares organisches Material enthaltend in der Masse eine Verbindung der Formel I gemäss Anspruch 1 als Fluoreszenzfarbstoff.

11. Hochmolekulares organisches Material enthaltend in der Masse ein in situ durch thermischen Abbau einer löslichen Verbindung der Formel I gemäss Anspruch 1 erzeugtes Pigment der Formel A(H)ₓ, worin A und x die in Anspruch 1 angegebene Bedeutung haben.

12. Thermo-, photo-. oder chemosensitives Aufzeichnungsmaterial enthaltend eine Verbindung der Formel I gemäss Anspruch 1.

13. Photo- und elektrolumineszente Materialien enthaltend eine Verbindung der Formel I gemäss Anspruch 1.

14. Verfahren zur Kristallmodifikationsumwandlung von chromophoren der Formel
A(H)ₓ (XVII)
durch Umsetzung in Verbindungen der Formel I gemäss Anspruch 1 und thermische Behandlung bei Temperaturen zwischen 100 und 170°C der erhaltenen Verbindungen der Formel I.

## Revendications

1. Composés de formule
A(B)ₓ (I),
dans laquelle
x est un nombre entier compris entre 1 et 4,
A représente le reste d'un chromophore de la famille de la quinacridone, de l'anthraquinone, du pérylène, de l'indigo, de la quinophtalone, de l'isoindolinone, de l'isoindoline, de la dioxazine, de la phtalocyanine, du dicétopyrrolopyrrole ou azoïque, lequel reste présente x atomes de N liés à B, de préférence avec au moins un groupe carbonyle directement adjacent ou conjugué,
B représente un groupe de formule 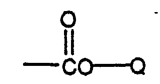
et lorsque x vaut 2, 3 ou 4, peut représenter aussi une, deux ou trois fois l'atome d'hydrogène, Q étant un groupe de formule 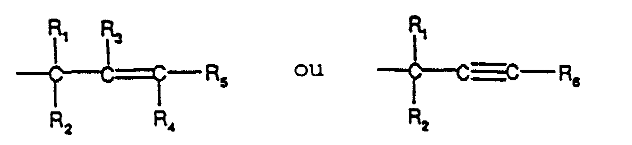 où
R₁ et R₂ représentent, indépendamment l'un de l'autre, un atome d'hydrogène, des groupes alkyle en C₁-C₂₄, cycloalcényle en C₄-C₁₂, cycloalkyle en C₄-C₁₂, alcynyle en C₃-C₂₄, alcényle en C₃-C₂₄, alkyle en C₁-C₂₄ interrompu par des groupes O, S ou N(R₉)₂, phényle ou biphényle non substitué ou substitué par des substituants alkyle en C₁-C₆, alkoxy en C₁-C₆, halogène, cyano ou nitro,
R₃, R₄ et R₅ représentent, indépendamment l'un de l'autre, des atomes d'hydrogène, des groupes alkyle en C₁-C₂₄ ou alcényle en C₃-C₂₄,
R₆ représente un atome d'hydrogène, des groupes alkyle en C₁-C₂₄, alcényle en C₃-C₂₄ ou un groupe de formule 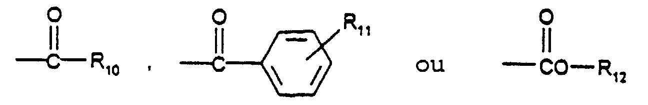
R₉ et R₁₀ représentent des groupes alkyle en C₁-C₆,
R₁₁ représente un atome d'hydrogène ou un groupe alkyle en C₁-C₆, et
R₁₂ représente un atome d'hydrogène, des groupes alkyle en C₁-C₆, phényle non substitué ou substitué par des substituants alkyle en C₁-C₆,
à la condition que lorsque A représente le reste d'un chromophore de la famille de dïcétopyrrolopyrrole, Q ne représente pas un groupe de formule

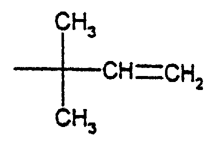

2. Composés selon la revendication 1 de formule I, où
R₁ représente des groupes alkyle en C₁-C₂₄, alcényle en C₃-C₂₄, alcynyle en, C₃-C₂₄, phényle non substitué ou substitué par des substituants alkyle en C₁-C₆, alkoxy en C₁-C₆, halogène, cyano ou nitro et
R₆ représente des groupes alkyle en C₁-C₂₄ ou alcényle en C₃-C₂₄.

3. Composés selon la revendication 1, de formule I, où
R₁ et R₂ représentent des groupes méthyle,
R₃, R₄ et R₅, représentent, indépendamment les uns des autres, des atomes d'hydrogène ou des groupes alkyle en C₁-C₁₂,
R₆ représente un groupe alkyle en C₁-C₆.

4. Composés selon la revendication 3, de formule I, où
B représente un groupe de formule 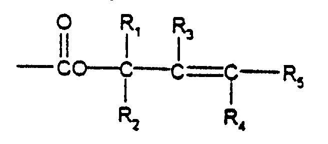
R₁ et R₂ représentent des groupes méthyle,
R₃, R₄ et R₅ représentent, indépendamment les uns des autres, des atomes d'hydrogène ou des groupes alkyle en C₁-C₁₂.

5. Composés selon la revendication 1 pris dans le groupe constitué par des
Des composés préférés de formule I sont :
a) imides de l'acide pérylène carboxylique de formule 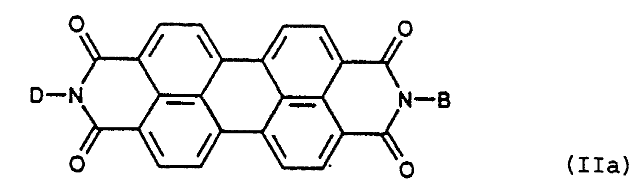 ou 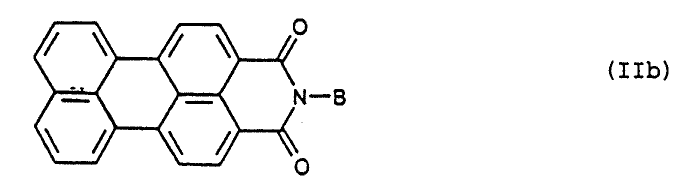 où D représente un atome d'hydrogène, un groupe alkyle en C₁-C₆, des groupes phénéthyle, benzyle ou phényle non substitué ou substitué par des atomes d'halogène ou des groupes alkyle en C₁-C₆, ou représente B,
b) quinacridones de formule 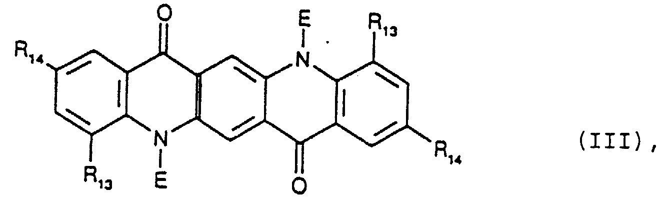 où R₁₃ et R₁₄ représentent, indépendamment l'un de l'autre, des atomes d'hydrogène, des atomes d'halogènes, des groupes alkyle en C₁-C₂₄, alkoxy en C₁-C₆ ou phényle,
c) dioxazines de formule 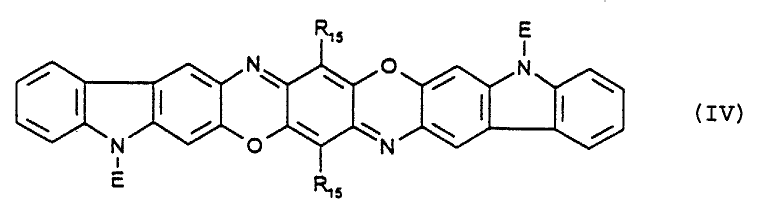 où R₁₅ représentent des atomes d'hydrogène ou des groupes alkyle en C₁-C₂₄,
ou de formule 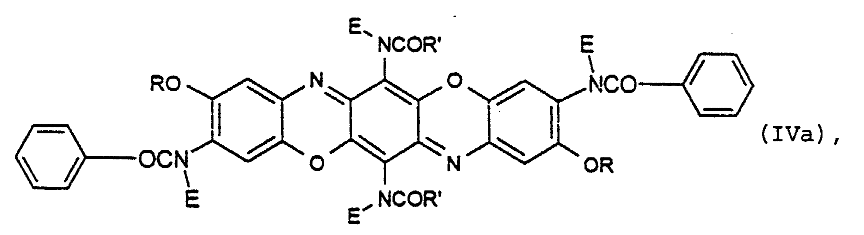 où R et R' représentent, indépendamment l'un de l'autre, des groupes alkyle en C₁-C₄,
d) isoindolines de formules 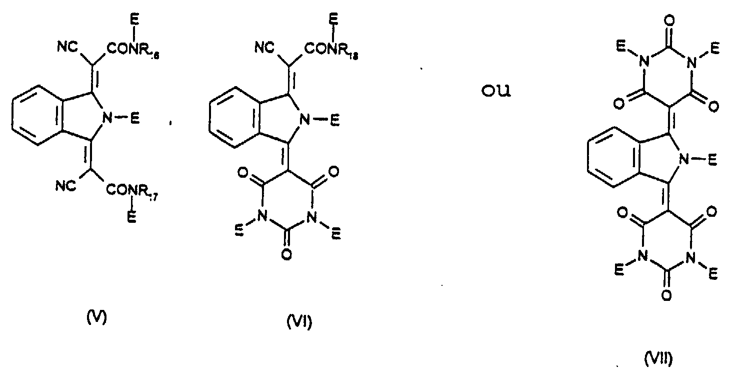 où
R₁₆ représente un groupe 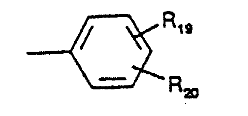
R₁₇ représente un atome d'hydrogène, des groupes alkyle en C₁-C₂₄, benzyle ou un groupe 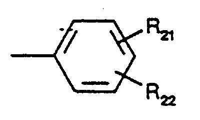
R₁₈ possède la même signification que R₁₆,
R₁₉, R₂₀, R₂₁ et R₂₂ représentent, indépendamment les uns des autres, des atomes d'hydrogène, des groupes alkyle en C₁-C₂₄, alkoxy en C₁-C₆, des atomes d'halogènes ou des groupes trifluorométhyle,
e) dérivés d'indigo de formule 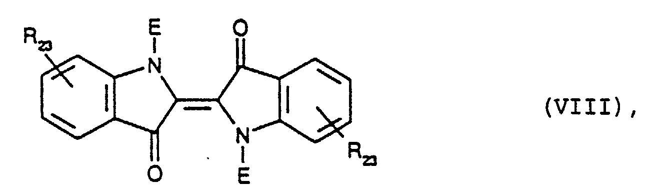 où
R₂₃ représentent des atomes d'hydrogène, des groupes CN, alkyle en C₁-C₆, alkoxy en C₁-C₆ ou des atomes d'halogènes,
f) composés benzimidazolone-azoïques de formule 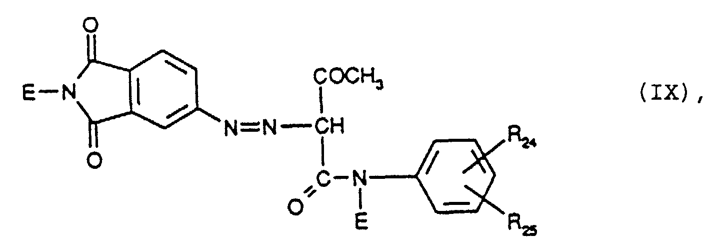 où R₂₄ et R₂₅ représentent, indépendamment l'un de l'autre, des atomes d'hydrogène, des atomes d'halogène, des groupes alkyle en C₁-C₆ ou alkoxy en C₁-C₆,
g) composés d'anthraquinoïdes de formule 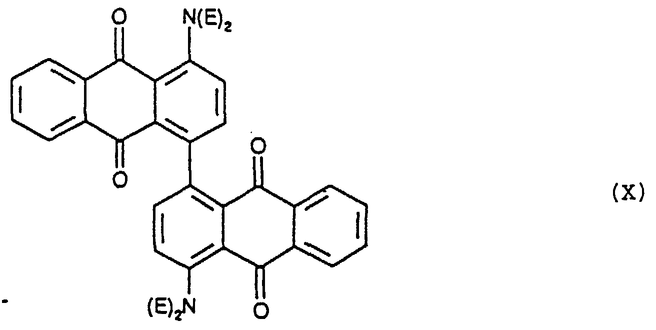 ou 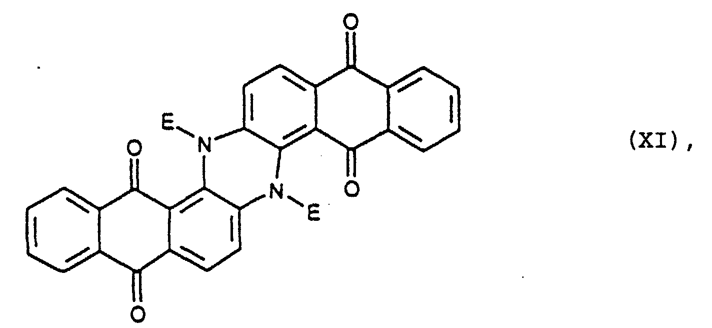
h) phtalocyanines de formule 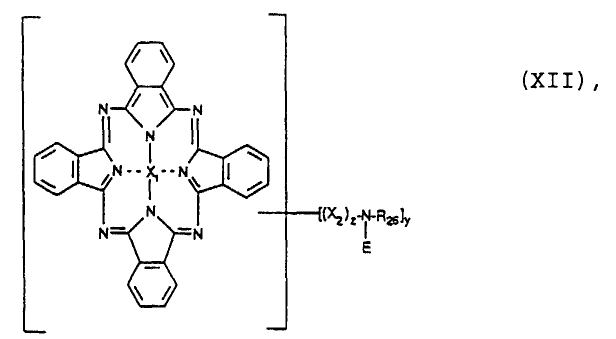 où
X₁ représente H₂, un métal bivalent pris dans le groupe comportant Cu(II), Zn(II), Fe(II), Ni(II), Ru(II), Rh(II), Pd(II), Pt(II), Mn(II), Mg(II), Be(II), Ca(II), Ba(II), Cd(II), Hg(II), Sn(II), Co(II) ou Pb(II), ou un oxométal bivalent pris dans le groupe comportant VO, MnO, TiO,
X₂ représente -CH(R₂₄)-, -CO- ou -SO₂-
R₂₆ représente un atome d'hydrogène, des groupes alkyle en C₁-C₆, -N(E)R₂₇, -NHCOR₂₈, -COR₂₈ ou 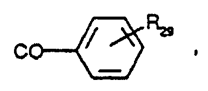
R₂₇ représente un atome d'hydrogène ou des groupes alkyle en C₁-C₆,
R₂₈ représente un groupe alkyle en C₁-C₆ et
R₂₉ représente un atome d'hydrogène, un atome d'halogène, des groupes alkyle en C₁-C₆ ou alkoxy en C₁-C₆,
z vaut zéro ou 1 et
y va de 1 à 4,
i) isoindolinones de formule 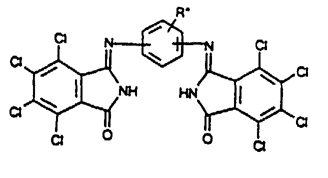 où R" représente un atome d'hydrogène ou un groupe alkyle en C₁-C₄, et
j) pyrrolo[3,4-c]pyrroles de formule 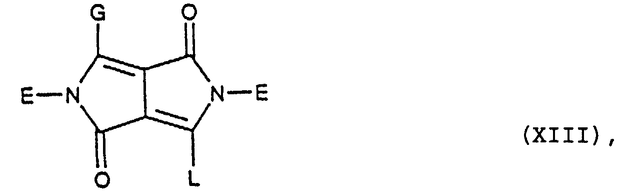 dans laquelle G et L représentent, indépendamment l'un de l'autre, un groupe de formule 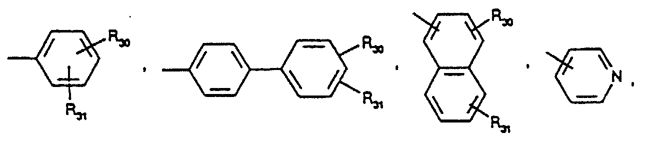 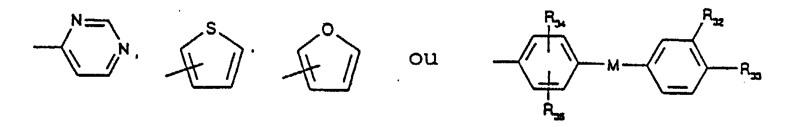 où
R₃₀ et R₃₁ représentent, indépendamment l'un de l'autre, des atomes d'hydrogène, des atomes d'halogènes, des groupes alkyle en C₁-C₂₄, alkoxy en C₁-C₆, (alkyl en C₁-C₁₈)mercapto, (alkyl en C₁-C₁₈)amino, -CN, -NO₂, -phényle, trifluorométhyle, cycloalkyle en C₅-C₆, -C=N-(alkyle en C₁-C₂₄), 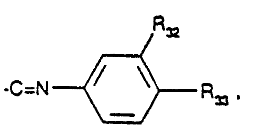 imidazolyle, pyrrazolyle, triazolyle, pipérazinyle, oxazolyle, bezoxazolyle, benzthioazolyle, benzimidazolyle, morpholinyle, pipéridinyle ou pyrrolidinyle,
M représente -CH₂-, -CH(CH₃)-, -CH(CH₃)₂-, -CH=N-, -N=N-, -O-, -S-, -SO-, -SO₂- ou -NR₃₆-,
R₃₂ et R₃₃ représentent, indépendamment l'un de l'autre, des atomes d'hydrogène, des atomes d'halogènes, des groupes alkyle en C₁-C₆, alkoxy en C₁-C₆ ou -CN,
R₃₄ et R₃₅ représentent, indépendamment l'un de l'autre, des atomes d'hydrogène, des atomes d'halogènes, des groupes alkyle en C₁-C₆ et
R₃₆ représente un atome d'hydrogène ou des atomes d'hydrogène, un groupe alkyle en C₁-C₆,
dans les formules précitées, E représentant à chaque fois un atome d'hydrogène ou B, à la condition que, dans chaque formule, E soit au moins une fois B, et que B possède la signification donnée ci-dessus, à laquelle s'appliquent les préférences citées ci-dessus.

6. Composés selon la revendication 5 pris dans le groupe comportant des
quinacridones de formule III, où R₁₃ et R₁₄ représentent, indépendamment l'un de l'autre, des atomes d'hydrogène, des atomes de chlore ou des groupes méthyle, ou
pyrrolo[3,4-c]pyrroles de formule XIII, où G et L sont identiques et représentent un groupe de formule 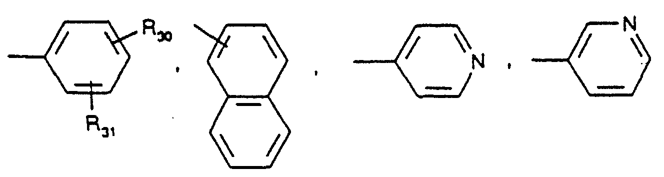 ou 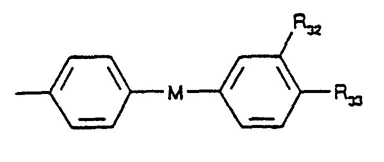 où
R₃₀ et R₃₁ représentent, indépendamment l'un de l'autre, des atomes d'hydrogène, de chlore, de brome, des groupes alkyle en C₁-C₄, alkoxy en C₁-C₆, (alkyl en C₁-C₆)amino, CN ou phényle,
M représente -O-, -NR₃₆-, -N=N- ou -SO₂-,
R₃₂ et R₃₃ représentent des atomes d'hydrogène, et
R₃₆ représente un atome d'hydrogène, des groupes méthyle ou éthyle, ou
des composés d'anthraquinoïdes de formule IX,
E dans les formules III, XIII et XI possédant à chaque fois la signification donnée à la revendication 5.

7. Composés selon la revendication 4 de formules 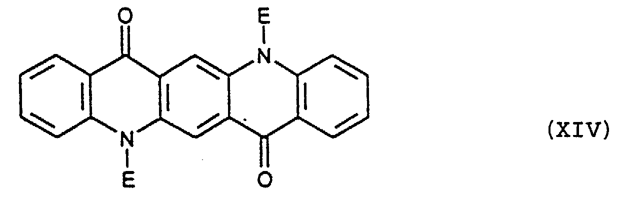 ou 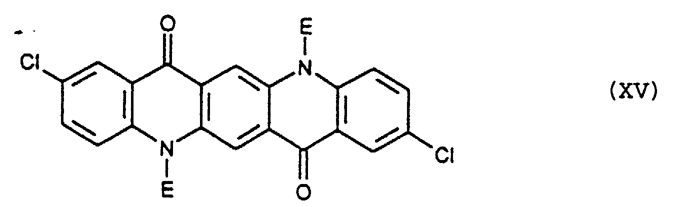 où
E représente des atomes d'hydrogène ou B, à la condition que E dans chacune des formule soit au moins une fois B, et B possède la signification donnée à la revendication 4.

8. Composés selon la revendication 4 de formule 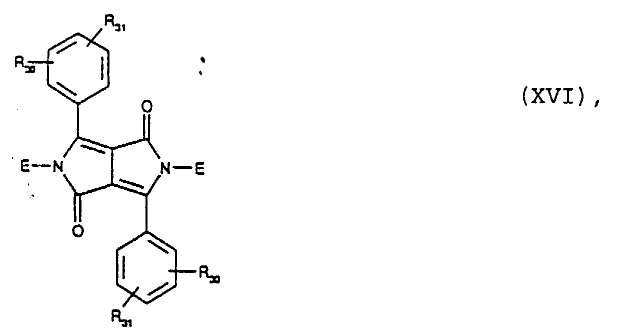 où
R₃₀ et R₃₁ représentent, indépendamment l'un de l'autre, des atomes d'hydrogène, des groupes méthyle, tert.-butyle, chloro, bromo, CN ou phényle, et
E représentent des atomes d'hydrogène ou B, à la condition que E soit au moins une fois B et que B possède la signification donnée à la revendication 4.

9. Composés selon la revendication 4, de formule 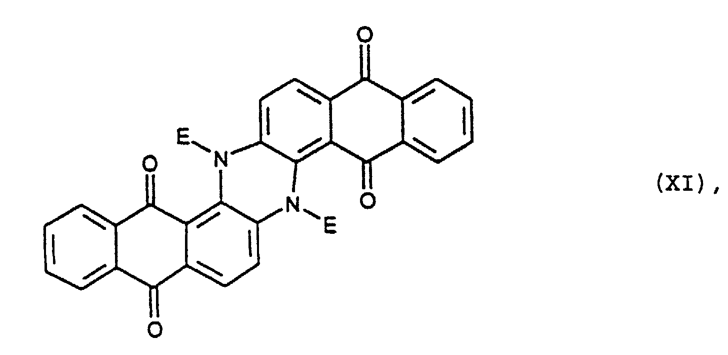 où E représente des atomes d'hydrogène ou B, à la condition que E soit au moins une fois B, et que B possède la signification donnée à la revendication 4.

10. Matière organique de haut poids moléculaire contenant dans la masse un composé de formule I selon la revendication 1 en tant que colorant fluorescent.

11. Matière organique de haut poids moléculaire contenant dans la masse un pigment de formule A(H)x où A et x possèdent les significations données à la revendication 1, ledit pigment étant formé in situ par dégradation thermique d'un composé soluble de formule I selon la revendication 1.

12. Matière d'enregistrement thermo-, photo- ou chimiosensible contenant un composés de formule I selon la revendication 1.

13. Matière photo- et électroluminescente contenant un composé de formule I selon la revendication 1.

14. Procédé pour la transformation de modification cristalline des chromophores de formule
A(H)ₓ (XVII)
par transformation en composés de formule I selon la revendication 1 et par traitement thermique à une température comprise entre 100 et 170°C des composés de formule I obtenus.

## Claims

1. A compound of formula
A(B)ₓ (I),
wherein x is an integer from 1 to 4,
A is the radical of a chromophore of the quinacridone, anthraquinone, perylene, indigo, quinophthalone, isoindolinone, isoindoline, dioxazine, phthalocyanine, diketopyrrolopyrrole or azo series, which radical A contains x N-atoms linked to B, preferably with at least one directly adjacent or conjugated carbonyl group,
B is a group of formula 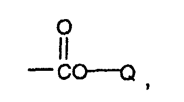 and, if x = 2, 3 or 4, can also be one, two or three hydrogen atom(s), and wherein Q is a group of formula 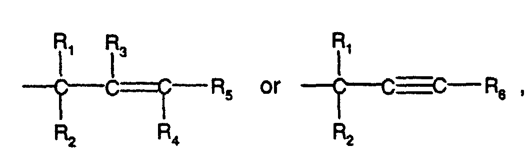 wherein
R₁ and R₂ are each independently of the other hydrogen, C₁-C₂₄alkyl; C₁-C₂₄alkyl, C₃-C₂₄alkenyl, C₃-C₂₄alkynyl, C₄-C₁₂cycloalkyl, C₄-C₁₂cyclcalkenyl, each of which is interrupted by O, S or N(R₉)₂; phenyl or biphenyl, each of which is unsubstituted or substituted by C₁-C₆alkyl, C₁-C₆alkoxy, halogen, cyano or nitro, R₃, R₄ and R₅ are each independently of one another hydrogen, C₁-C₂₄alkyl or C₃-C₂₄alkenyl,
R₆ is hydrogen, C₁-C₂₄alkyl, C₃-C₂₄alkenyl or a group of formula 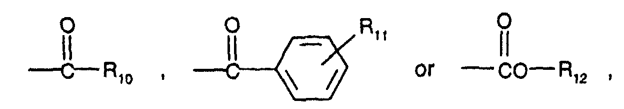
R₉ and R₁₀ are C₁-C₆alkyl, R₁₁ is hydrogen or C₁-C₆alkyl, and R₁₂ is hydrogen, C₁-C₆alkyl, unsubstituted or C₁-C₆alkyl-substituted phenyl,
with the proviso that, if A is the radical of a chromophore of the diketopyrrolopyrrole series, then Q is not a group of formula

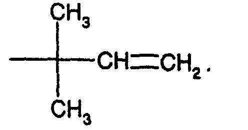

2. A compound according to claim 1 of formula I, wherein R₁ is C₁-C₂₄alkyl, C₃-C₂₄alkenyl, C₃-C₂₄alkynyl, phenyl which is unsubstituted or substituted by C₁-C₆alkyl, C₁-C₆alkoxy, halogen, cyano or nitro, and R₆ is C₁-C₂₄alkyl or C₃-C₂₄alkenyl.

3. A compound according to claim 1 of formula I, wherein
R₁ and R₂ are methyl,
R₃, R₄ and R₅ are each independently of one another hydrogen or C₁-C₁₂alkyl,
R₆ is C₁-C₆alkyl.

4. A compound according to claim 3 of formula I, wherein
B is a group of formula 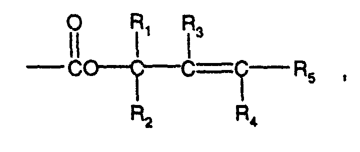
R₁ and R₂ are methyl, and R₃, R₄ and R₅ are each independently of one another hydrogen or C₁-C₁₂alkyl.

5. A compound according to claim 1 selected from the group consisting of
a) perylenecarboximides of formula 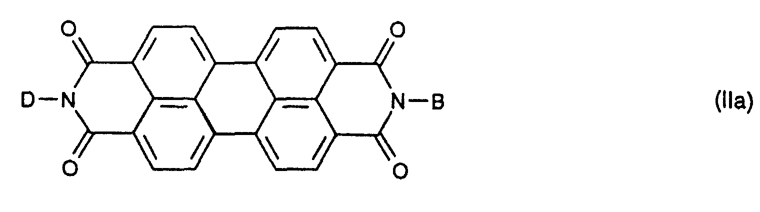 or 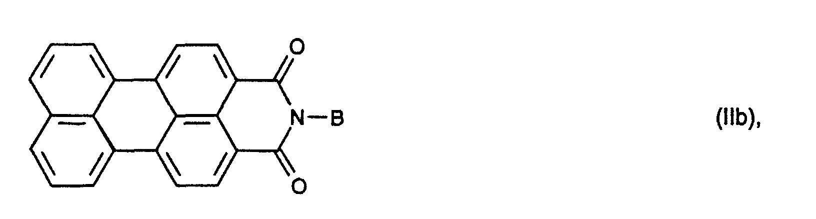 wherein D is hydrogen, C₁-C₆alkyl; unsubstituted or halogen- or C₁-C₆alkyl-substituted phenyl, benzyl or phenethyl, or B,
b) quinacridones of formula 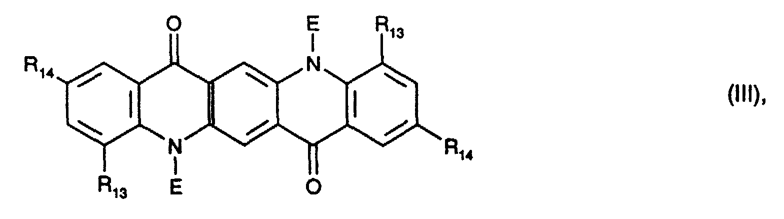 wherein R₁₃ and R₁₄ are each independently of the other hydrogen, halogen, C₁-C₂₄alkyl, C₁-C₆alkoxy or phenyl,
c) dioxazines of formula 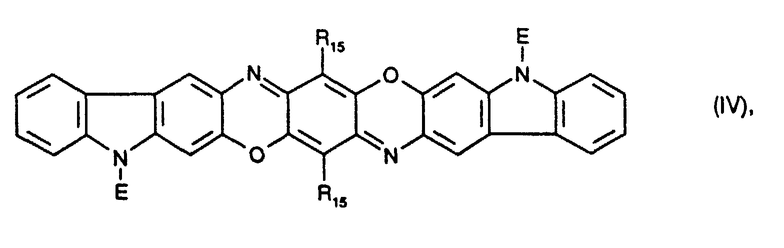 wherein R₁₅ is hydrogen, halogen or C₁-C₂₄alkyl, or of formula 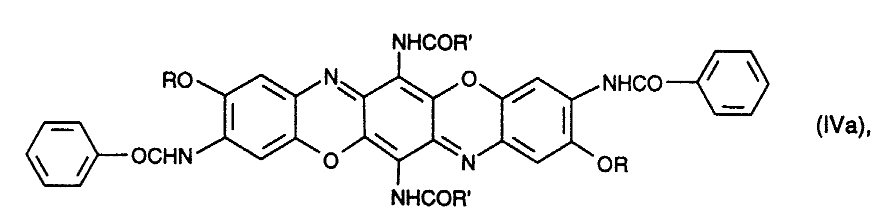 wherein R and R' are each independently of the other C₁-C₄alkyl,
d) isoindolines of formulae 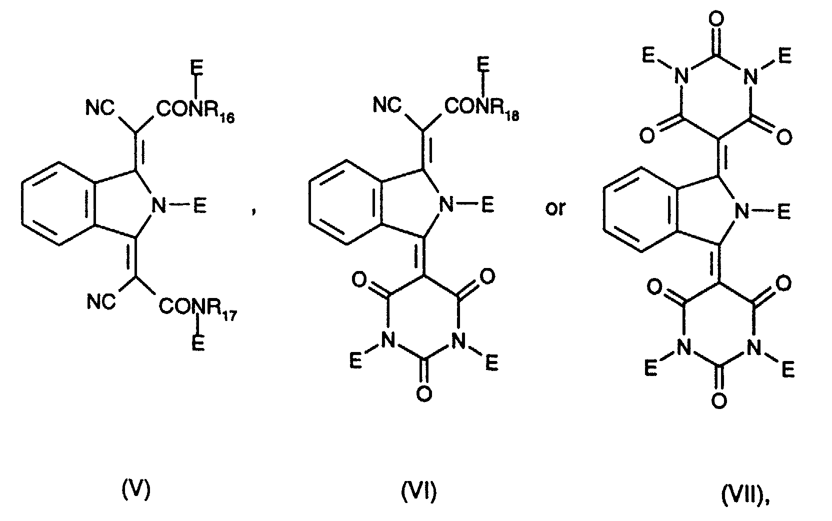
wherein R₁₆ is a group of formula 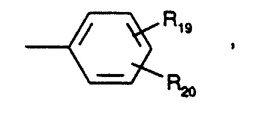
R₁₇ is hydrogen, C₁-C₂₄alkyl, benzyl or a group of formula 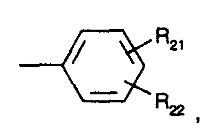
R₁₈ has the same meaning as R₁₆,
R₁₉, R₂₀, R₂₁ and R₂₂ are each independently of one another hydrogen, C₁-C₂₄alkyl, C₁-C₆alkoxy, halogen or trifluoromethyl,
e) indigo derivatives of formula 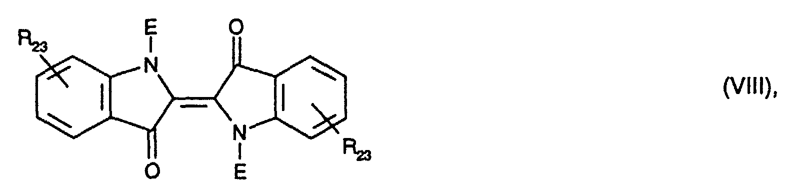 wherein R₂₃ is hydrogen, CN, C₁-C₆alkyl, C₁-C₆alkoxy or halogen,
f) azobenzimidazolones of formula 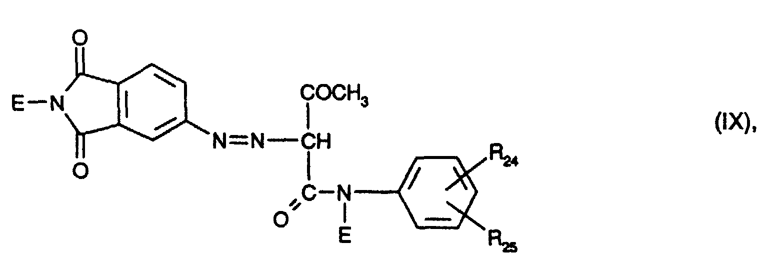 wherein R₂₄ and R₂₅ are each independently of the other hydrogen, halogen, C₁-C₆alkyl or C₁-C₆alkoxy,
g) anthraquinoid compounds of formula 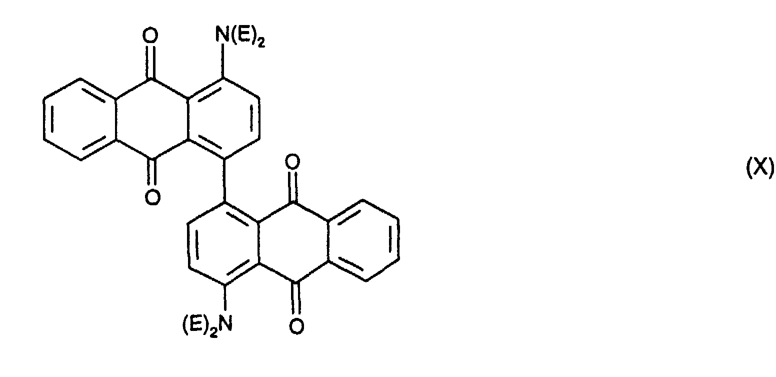 or 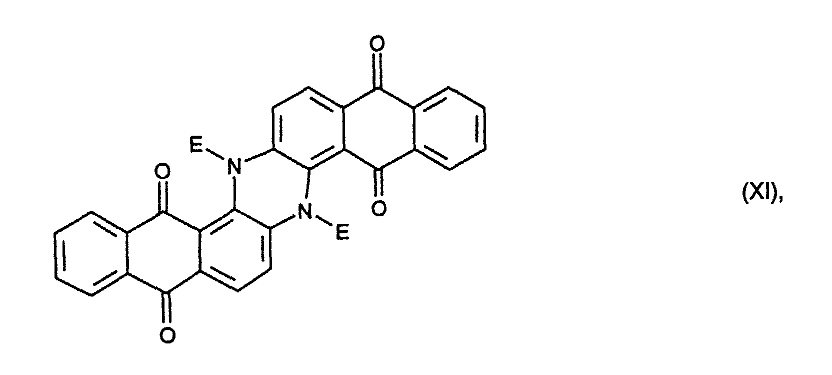
h) phthalocyanines of formula 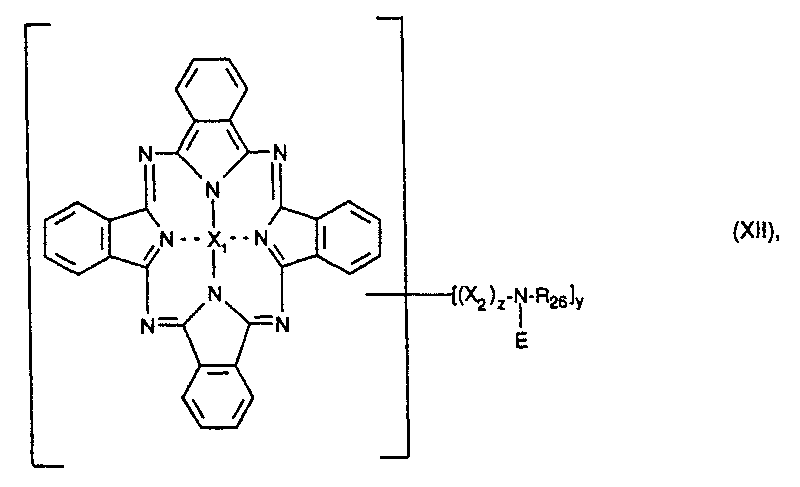 wherein
X₁ is H₂, a divalent metal selected from the group consisting of Cu(II), Zn(II), Fe(II), Ni(II), Ru(II), Rh(II), Pd(II), Pt(II), Mn(II), Mg(II), Be(II), Ca(II), Ba(II), Cd(II), Hg(II), Sn(II), Co(II) and Pb(II), or a divalent oxo metal selected from the group consisting of V(O), Mn(O) and TiO,
X₂ is -CH(R₂₄)-, -CO- or -SO₂-,
R₂₆ is hydrogen, C₁-C₆alkyl, -N(E)R₂₇, -NHCOR₂₈, -COR₂₈ or 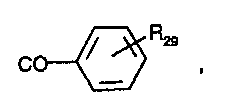
R₂₇ is hydrogen or C₁-C₆alkyl, R₂₈ is C₁-C₈alkyl, and R₂₉ is hydrogen, halogen, C₁-C₆alkyl or C₁-C₆alkoxy,
z is 0 or 1, and y is an integer from 1 to 4,
i) isoindolinones of formula 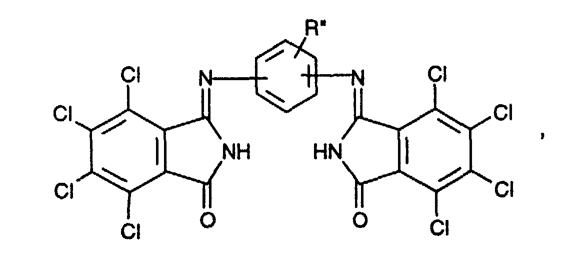 wherein R" is hydrogen or C₁-C₄alkyl,
and
j) pyrrolo[3,4-c]pyrroles of formula 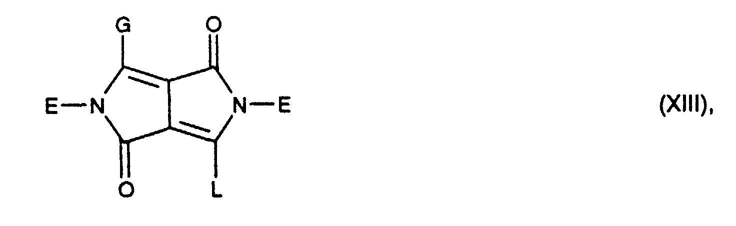
wherein G and L are each independently of the other a group of formula 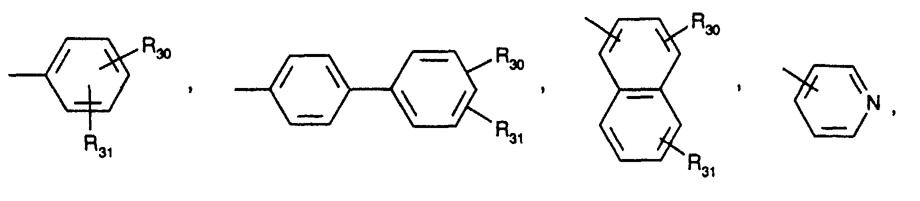 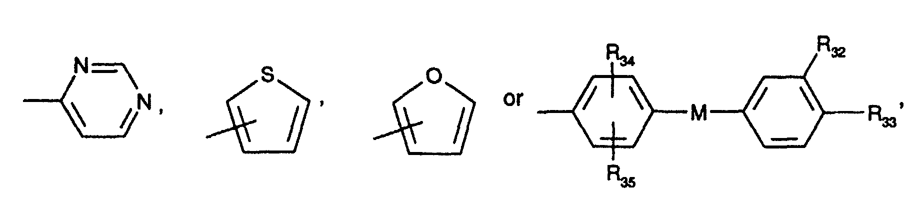 wherein
R₃₀ and R₃₁ are each independently of the other hydrogen, halogen, C₁-C₂₄alkyl, C₁-C₆alkoxy, C₁-C₁₈alkylmercapto, C₁-C₁₈alkylamino, -CN, -NO₂, phenyl, trifluoromethyl, C₅-C₆cycloalkyl, -C=N-(C₁-C₂₄alkyl), 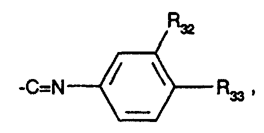 imidazolyl, pyrrazolyl, triazolyl, piperazinyl, pyrrolyl, oxazolyl, benzoxazolyl, benzthiazolyl, benzimidazolyl, morpholinyl, piperidinyl or pyrrolidinyl,
M is -CH₂-, -CH(CH₃)-, -C(CH₃)₂-, -CH=N-, -N=N-, -O-, -S-, -SO-, -SO₂- or -NR₃₆-,
R₃₂ and R₃₃ are each independently of the other hydrogen, halogen, C₁-C₆alkyl, C₁-C₆alkoxy or -CN, R₃₄ and R₃₅ are each independently of the other hydrogen, halogen or C₁-C₆alkyl, and
R₃₆ is hydrogen or C₁-C₆alkyl,
E in the above formulae being hydrogen or B, with the proviso that E in each formula is at least once B, and B is as claimed in claim 1.

6. A compound according to claim 5 selected from the group consisting of quinacridones of formula III, wherein R₁₃ and R₁₄ are each independently of the other hydrogen, chloro or methyl, and of pyrrolo[3,4-c]pyrroles of formula XIII, wherein G and L are identical and are a group of formula 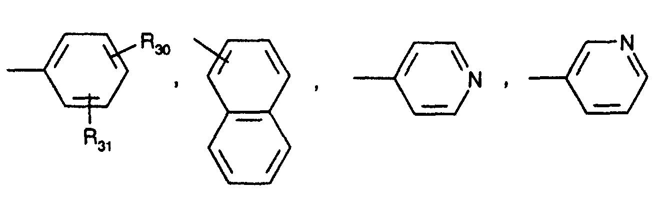 or 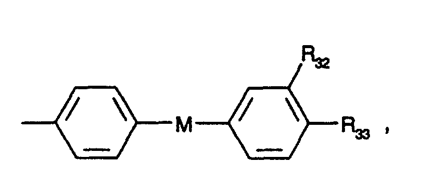
wherein R₃₀ and R₃₁ are each independently of the other hydrogen, chloro, bromo, C₁-C₄alkyl, C₁-C₆alkoxy, C₁-C₆alkylamino, CN or phenyl,
M is -O-, -NR₃₆-, -N=N- or -SO₂-,
R₃₂ and R₃₃ are hydrogen, and R₃₆ is hydrogen, methyl or ethyl, and
of anthraquinoid compounds of formula XI,
wherein E in formulae III, XIII and XI is as claimed in claim 5.

7. A compound according to claim 4, of formula 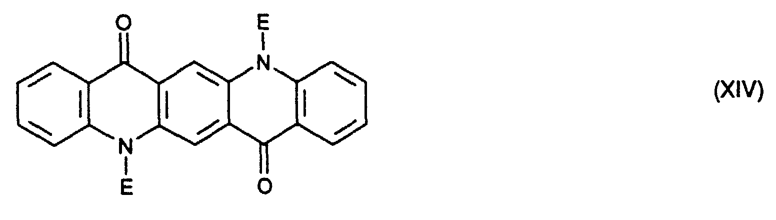 or 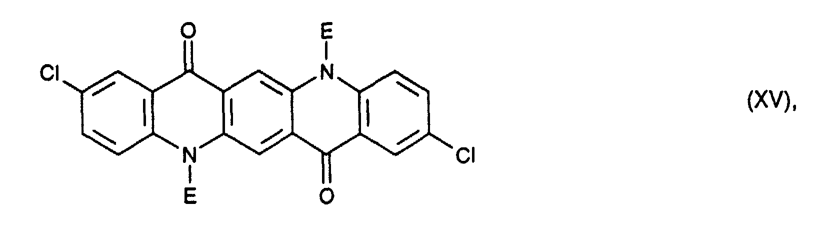 wherein E is hydrogen or B, with the proviso that E in each formula is at least once B, and B is as claimed in claim 4.

8. A compound according to claim 4, of formula 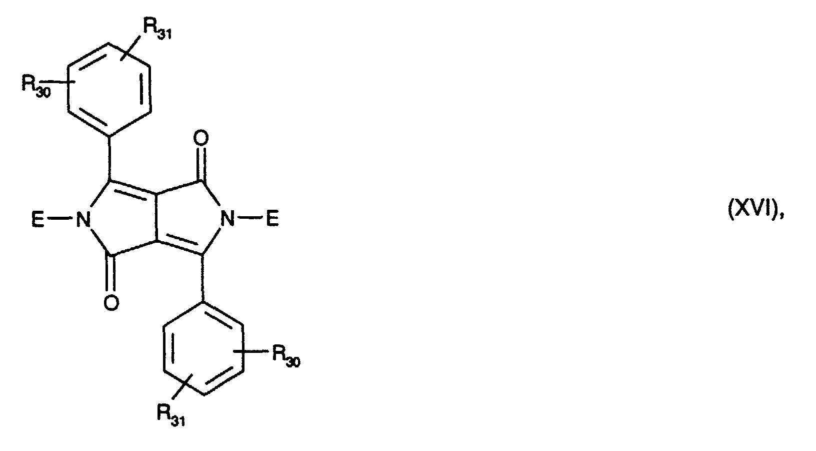 wherein R₃₀ and R₃₁ are each independently of the other hydrogen, methyl, tert-butyl, chloro, bromo, CN or phenyl, and
E is hydrogen or B, with the proviso that E is at least once B, and B is as claimed in claim 4.

9. A compound according to claim 4, of formula 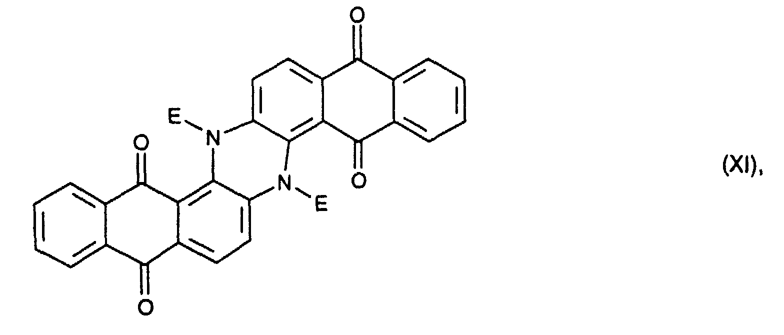 wherein E is hydrogen or B, with the proviso that E is at least once B, and B is as claimed in claim 4.

10. High molecular weight organic material comprising in the mass a compound of formula I according to claim 1 as fluorescent pigment.

11. High molecular weight organic material containing in the mass a pigment of formula A(H)ₓ, wherein A and x are as claimed in claim 1, produced in situ by thermal degradation of a soluble compound of formula I according to claim 1.

12. Thermosensitive, photosensitive or chemosensitive recording material comprising a compound of formula I according to claim 1.

13. Photo- and electroluminescent material comprising a compound of formula I according to claim 1.

14. A process for the crystal modification conversion of chromophores of formula
A(H)ₓ (XVII)
by conversion to compounds of formula I according to claim 1 and by thermal treatment of the resulting compounds of formula I in the temperature range from 100 to 170°C.
